# EUROPÄISCHE PATENTSCHRIFT

(11) **EP 2 069 413 B1**
(45) Veröffentlichungstag und Bekanntmachung des Hinweises auf die Patenterteilung: **03.08.2011**
(21) Anmeldenummer: 07820930.1
(22) Anmeldetag: 04.10.2007
(51) Int. Cl.: C08F 290/06, C08F 220/18, C08F 220/20, C08F 222/12, A01N 25/10, A61K 47/32

(54) **KAMMPOLYMERE ENTHALTENDE WIRKSTOFFFORMULIERUNGEN**
ACTIVE INGREDIENT FORMULATIONS CONTAINING COMB POLYMERS
FORMULATIONS DE SUBSTANCE ACTIVE CONTENANT DES POLYMÈRES EN PEIGNE

(30) Priorität: 05.10.2006 EP 06121851
(43) Veröffentlichungstag der Anmeldung: 17.06.2009
(73) Patentinhaber: BASF SE, 67056 Ludwigshafen (DE)
(72) Erfinder: KOLTZENBURG, Sebastian, 67125 Dannstadt-Schauernheim (DE); DOMBO, Peter, 65207 Wiesbaden (DE); OETTER, Günter, 67227 Frankenthal (DE); BRATZ, Matthias, 67133 Maxdorf (DE)
(86) Internationale Anmeldenummer: PCT/EP2007/060553
(87) Internationale Veröffentlichungsnummer: WO 2008/040786

(56) Entgegenhaltungen:
- WO-A-03/086493
- WO-A-2004/100665
- US-A- 5 468 821
- US-B1- 6 248 805
- DATABASE WPI Week 200639 Derwent Publications Ltd., London, GB; AN 2006-380961 XP002465957 & WO 2006/051746 A (SHISEIDO CO LTD) 18. Mai 2006 (2006-05-18)

## Beschreibung

Die vorliegende Erfindung betrifft Wirkstoffformulierungen enthaltend Kammpolymere. Die Kammpolymere werden zur Stabilisierung von in Wasser nicht oder schlecht löslichen Wirkstoffen in einer wässrigen Phase verwendet. Im Wasser nicht lösliche Wirkstoffe sind insbesondere Wirkstoffe für den Pflanzenschutz.

Wirkstoffe, d. h. Substanzen, die auch in geringer Konzentration bereits eine physiologische Wirkung entfalten können, werden häufig in Form wässriger Wirkstoffaufbereitungen angewendet. So werden beispielsweise im Pflanzenschutz die zur Schädlingsbekämpfung eingesetzten Wirkstoffe, d. h. Pflanzenschutzwirkstoffe wie Insektizide, Akarizide, Nematizide, Fungizide und Herbizide, aber auch Wachstumsregulatoren, häufig als Konzentrate, z. B. als wässrige Konzentrate wie Suspensionen oder Emulsionen, aber auch als feste Konzentrate wie Pulver, Stäube oder Granulate, formuliert und vertrieben. Diese Formulierungen werden in der Regel vor ihrer Anwendung durch Zugabe einer großen Menge Wasser auf die gewünschte Anwendungskonzentration verdünnt (so genannte "Spritzbrühe"). Auch für pharmazeutisch und kosmetisch aktive Substanzen sowie für Nahrungsmittelzusätze, z. B. Vitamine, Provitamine etc., haben sich Formulierungen bewährt, die eine Stabilisierung oder Solubilisierung des Wirkstoffs in einem wässrigen Medium, beispielsweise in einem flüssigen Nahrungsmittel oder in einer Infusionslösung, aber auch In Körperflüssigkeiten, ermöglichen.

Ein prinzipielles Problem stellt die in der Regel geringe Wasserlöslichkeit der Wirkstoffe dar, die häufig weniger als 10 g/l bei 23 °C/1013 mbar beträgt. Wässrige Formulierungen derartiger Wirkstoffe und ebenso wässrige gebrauchsfertige Aufbereitungen sind daher heterogene Systeme, in denen der Wirkstoff als emulgierte bzw. dispergierte Phase in einer kontinuierlichen wässrigen Phase vorliegt. Zur Stabilisierung dieser an sich metastabilen Systeme werden üblicherweise Emulgatoren oder Dispergierhilfsmittel eingesetzt. Deren stabilisierende Wirkung ist jedoch häufig nicht zufriedenstellend, so dass eine Abscheidung des Wirkstoffs, beispielsweise ein Aufrahmen oder ein Sedimentieren des Wirkstoffs auftreten kann, insbesondere wenn die wässrige Formulierung längere Zeit bei erhöhter Temperatur und/oder bei stark wechselnden Temperaturen oder in der Nähe des Gefrierpunkts gelagert wird. Dieses Problem ist insbesondere dann ausgeprägt, wenn der Wirkstoff zur Kristallisation neigt. Häufig kommt es auch zur Abscheidung von festen Wirkstoffpartikeln, wenn eine Formulierung, welche den Wirkstoff in konzentrierter Form enthält, mit Wasser verdünnt wird.

Häufig werden organische Lösungsmittel zur Herstellung von Formulierungen von in Wasser nicht löslichen Wirkstoffen eingesetzt. So verwendet man häufig mit Wasser mischbare Lösungsmittel als Löslichkeitsvermittler, d. h. zur Erhöhung der Löslichkeit des Wirkstoffs in der wässrigen Phase. Mit Wasser nicht mischbare Lösungsmittel wiederum dienen dazu, einen bei Anwendungstemperatur festen Wirkstoff in eine flüssige Phase zu überführen, die man dann leichter emulgieren kann. Im Unterschied zu Suspensionen des festen Wirkstoffs ist in den Emulsionen der Wirkstoff in den Lösungsmitteltröpfchen molekular gelöst und ist daher bei Applikation besser verfügbar und häufig wirksamer. Der Einsatz größerer Mengen organischer Lösungsmittel ist jedoch auf Grund der bekannten VOC-Problematik aus arbeitshygienischen Gründen, Umweltaspekten und teilweise auch aus toxikologischen Gründen nicht wünschenswert.

Ein weiterer Nachteil konventioneller wässriger Wirkstoffformulierungen ist die vergleichsweise große Teilchengröße der in der wässrigen Phase suspendierten bzw. emulgierten Wirkstoffpartikel bzw. Wirkstofftröpfchen, die in der Regel mehrere µm beträgt. Wünschenswert sind wässrige Formulierungen, in denen der Wirkstoff in möglichst fein verteilter Form vorliegt oder beim Verdünnen mit Wasser in eine fein verteilte Form überführt wird, um einerseits eine gleichmäßige Verteilung in der Formulierung und damit eine bessere Handhabbarkeit und Dosierbarkeit zu gewährleisten und gleichzeitig, um die Bioverfügbarkeit des in der Formulierung bzw. in der gebrauchsfertigen Zusammensetzung enthaltenen Wirkstoffs zu erhöhen. Erstrebenswert sind dabei Formulierungen, die beim Verdünnen mit Wasser eine Wirkstoffaufbereitung liefern, in denen die den Wirkstoff enthaltende Phase mittlere Teilchengrößen unterhalb 500 nm und insbesondere unterhalb 300 nm aufweist.

Verschiedentlich wurde die Verwendung von amphiphilen Blockcopolymeren zur Solubilisierung bzw. Stabilisierung von in Wasser unlöslichen Wirkstoffen in einem wässrigen Vehikel vorgeschlagen (siehe z.B. WO2005/121201 und dort zitierte Literatur). Der Begriff "Solubilisierung" bezeichnet eine unter Verwendung von löslichkeitsvermittelnden Substanzen (Hilfsstoffen) erreichte stabile gleichmäßige Verteilung des wasserunlöslichen Wirkstoffs in der wässrigen Phase wobei die Partikel der dispersen Wirkstoffphase häufig so klein sind, dass sie sichtbares Licht kaum streuen und die Mischung daher mehr oder weniger transparent erscheint. Die amphiphilen Blockcopolymere weisen dabei in der Regel wenigstens einen hydrophilen Polymerblock und wenigstens einen hydrophoben Polymerblock auf. Die Herstellung der in WO 2005/121201 beschriebenen Blockcopolymere ist vergleichsweise aufwändig.

Die US 4,847,410 und US 4,959,156 beschreiben die Herstellung von Copolymeren aus Allylalkoholalkoxylaten und (Meth)acrylsäure und schlagen deren Einsatz als Dispergiermittel vor.

Die WO 03/043420 beschreibt die Verwendung von Copolymeren als Adjuvantien bei der Behandlung von Pflanzen. Die Copolymere bestehen aus Olefinen und/oder Vinylethern, sowie aus ethylenisch ungesättigten Dicarbonsäuren bzw. Dicarbonsäuredenvaten und weiteren Comonomeren.

Die DE 10338437 beschreibt Formulierungen von Agrowirkstoffen mit Adjuvantien auf Basis von Blockcopolymeren, die durch Umsetzung von (a) modifizierten Polyolefinen oder Oligoolefinen mit einer von ethylenisch ungesättigter Dicarbonsäure abgeleiteten Endgruppe mit (b) durch Homo- oder Co-Polymerisation von Oxiran- oder Aziridin-Derivaten erhaltenen Polymeren bereitgestellt werden.

Die WO 03/055944 beschreibt die Verwendung von Copolymeren auf Basis von Acrylamidomethylpropansulfonsäure (AMPS) als Kristallisationsinhibitor in wässrigen Suspensionskonzentraten für den Pflanzenschutz.

Aus der EP 1681923 sind Wirkstoffformulierungen bekannt, die wenigstens einen Wirkstoff und wenigstens ein statistisches Copolymer enthalten, das durch radikalische Polymerisation von olefinisch ungesättigten Sulfonsäuren mit Estern oder Amiden der Acrylsäure oder der Methacrylsäure erhältlich ist.

Die WO 06/000592 beschreibt die Verwendung von Ethergruppen enthaltenden Polymeren als Lösungsvermittler zur Herstellung von Pflanzenschutzmitteln, wobei die Polymere durch Copolymerisation mindestens eines hydrophoben Monomers und mindestens eines Allylalkoholalkoxylates erhältlich sind.

Die WO 2004/100665 beschreibt eine Zusammensetzung, die ein polymeres Biguanid, gegebenenfalls in Kombination mit weiteren antimikrobiellen Wirkstoffen, sowie ein basisches Copolymer enthält.

Die im Stand der Technik beschriebenen Polymere sind jedoch häufig bezüglich ihrer Solubilisierungseigenschaft für in Wasser schlecht lösliche oder unlösliche Wirkstoffe nicht zufriedenstellend oder ihre Herstellung ist aufwändig.

Der vorliegenden Erfindung liegt die Aufgabe zu Grunde, Wirkstoffformulierungen bereitzustellen, die eine effektive Solubilisierung von in Wasser unlöslichen oder schlecht löslichen Wirkstoffen in einem wässrigen Medium ermöglichen. Diese Wirkstoffformulierungen sollten insbesondere auch geeignet sein, eine Solubilisierung von in Wasser unlöslichen Wirkstofffen zu ermöglichen, die keinen oder nur einen sehr geringen Gehalt an flüchtigen organischen Substanzen aufweisen. Weiterhin ist eine hohe Stabilität der Wirkstoffformulierungen im Hinblick auf Entmischungsvorgänge bei langer Lagerung, bei Zusatz von Elektrolyt und beim Verdünnen mit Wasser wünschenswert.

Diese Aufgabe wird überraschenderweise durch Wirkstoffformulierungen enthaltend Kammpolymere gelöst, die durch Copolymerisation monoethylenisch ungesättigter Monomere M erhältlich sind, wobei die Monomere M:
a) wenigstens ein monoethylenisch ungesättigtes Monomer Ma, ausgewählt unter Estern monoethylenisch ungesättigter C₃-C₈-Monocarbonsäuren mit C₁-C₂₀-Alkanolen, C₅-C₁₀-Cycloalkanolen, Phenyl-C₁-C₄-alkanolen oder Phenoxy-C₁-C₄-alkanolen und den Diestern monoethylenisch ungesättigter C₄-C₈-Dicarbonsäuren mit C₁-C-₂₀-Alkanolen, C₅-C₁₀-Cycloalkanolen, Phenyl-C₁-C₄-alkanolen oder Phenoxy-C₁-C₄-alkanolen; und
b) wenigstens ein monoethylenisch ungesättigtes Monomer Mb, ausgewählt unter den Estern monoethylenisch ungesättigter C₃-C₈-Monocarbonsäuren und den Mono- und Diestern monoethylenisch ungesättigter C₄-C₈-Dicarbonsäuren mit Poly-(C₂-C₄-alkylenether)olen;
umfassen und wobei die Gesamtmenge der Monomere Ma und Mb wenigstens 80 Gew.-% der das Kammpolymer konstituierenden Monomere M ausmacht.

Die Kammpolymere sind in vorteilhafter Weise geeignet, Wirkstoffe, die in Wasser schlecht löslich oder unlöslich sind, in wässriger Phase zu stabilisieren und ermöglichen daher die Herstellung sowohl wässriger Formulierungen derartiger Wirkstoffe, als auch die Herstellung nicht-wässriger Formulierungen, die beim Verdünnen mit Wasser zu einer äußerst feinen Verteilung des Wirkstoffs in der wässrigen Phase führen. Durch die Kammpolymere und deren Verwendung lassen sich für die jeweilige Anwendung technisch relevante Mengen an Wirkstoff stabil solubilisieren, ohne dass hierzu organische Lösungsmittel benötigt werden.

Gegenstand der Erfindung sind daher Wirkformulierungen, die wenigstens einen in Wasser schlecht- bzw. unlöslichen Wirkstoff und wenigstens ein Kammpolymer, wie hier und im Folgenden beschrieben, enthalten.

Die Kammpolymeren werden zur Herstellung von wässrigen Aufbereitungen von Wirkstoffen, die in Wasser bei 25 °C/1013 mbar eine Löslichkeit unterhalb 10 g/l aufweisen, eingesetzt.

Gegenstand der vorliegenden Erfindung ist weiterhin ein Verfahren zur Herstellung wässriger Wirkstoffaufbereitungen, umfassend das Vermischen bzw. Verdünnen einer erfindungsgemäßen Wirkstoffformulierung mit Wasser.

Die erfindungsgemäßen Wirkstoff-Formulierungen liefern beim Verdünnen mit Wasser oder einer wässrigen Flüssigkeit wässrige Aufbereitungen des Wirkstoffs, umfassend eine wässrige, kontinuierliche Phase und wenigstens eine wirkstoffhaltige Phase mit mittleren Teilchengrößen deutlich unterhalb 1 µm, typischerweise nicht mehr 500 nm, häufig nicht mehr als 400 nm, insbesondere nicht mehr als 300 nm, besonders bevorzugt nicht mehr als 250 nm, 200 nm oder 150 nm und speziell nicht mehr als 100 nm z. B. im Bereich von 5 bis 500 nm oder 5 bis 400 nm, häufig im Bereich von 10 bis 300 nm, vorzugsweise im Bereich von 10 bis 250 nm, insbesondere im Bereich von 20 bis 200 nm oder 20 bis 150 nm und besonders bevorzugt im Bereich von 20 bis 100 nm. Je nach Art des Polymers und des Wirkstoffs sowie abhängig von den Konzentrationsverhältnissen können die Aggregate auch so klein werden, dass sie nicht mehr in Form nachweisbarer, diskreter Partikel vorliegen (Teilchengröße < 20 nm, < 10 nm oder < 5 nm).

Die hier und im Folgenden angegebenen Teilchengrößen sind gewichtsmittlere Teilchengrößen, wie sie durch dynamische Lichtstreuung ermittelt werden können. Verfahren hierzu sind dem Fachmann geläufig, beispielsweise aus H. Wiese in D. Distler, Wässrige Polymerdispersionen, Wiley-VCH 1999, Kapitel 4.2.1, S. 40ff und dort zitierte Literatur sowie H. Auweter, D. Horn, J. Colloid Interf. Sci. 105 (1985) 399, D. Lilge, D. Horn, Colloid Polym. Sci. 269 (1991) 704 oder H. Wiese, D. Horn, J. Chem. Phys. 94 (1991) 6429.

Wirkstoffe im Sinne der vorliegenden Erfindung sind chemisch definierte Substanzen, die in einem Organismus, meist bereits in kleinen Aufwandmengen, gezielt eine Wirkung bzw. eine Reaktion hervorrufen. Wirkstoffe im Sinne dieser Erfindung sind insbesondere organische Verbindungen mit einer definierten molekularen Zusammensetzung (Summenformel) und einem Molekulargewicht, dass typischerweise nicht mehr als 2000 Dalton, insbesondere nicht mehr als 1000 Dalton beträgt und vorzugsweise im Bereich von 100 bis 1000 Dalton und speziell im Bereich von 150 bis 500 Dalton liegt. Eine schlechte Löslichkeit bedeutet in diesem Zusammenhang eine Löslichkeit des Wirkstoffs in Wasser von unterhalb 10 g/l, häufig unterhalb 5 g/l und insbesondere unterhalb 1 g/l und speziell unterhalb 0,1 g/l bei 25 °C und 1013 mbar.

Die erfindungsgemäßen Wirkformulierungen können fest, pastös oder flüssig sein.

Die Begriffe "wässriges Medium" und "wässrige Phase" umfassen hier und im Folgenden Wasser, wässrige Mischungen von Wasser mit bis zu 10 Gew.-%, bezogen auf die Mischung, an organischen Lösungsmitteln, die mit Wasser mischbar sind, und Lösungen von Feststoffen in Wasser oder in den wässrigen Mischungen. Beispiele für mit Wasser mischbare Lösungsmittel umfassen C₃-C₄-Ketone wie Aceton und Methylethylketon, cyclische Ether wie Dioxan und Tetrahydrofuran, C₁-C₄-Alkanole wie Methanol, Ethanol, n-Propanol, Isopropanol, n-Butanol, tert.-Butanol, Polyole und deren Mono- und Dimethylether wie Glykol, Propandiol, Ethylenglykolmonomethylether, Diethylenglykol, Diethylenglykolmonomethylether. Diethylenglykoldimethylether, Glycerin, weiterhin C₂-C₃-Nitrile wie Acetonitril und Propionitril, Dimethylsulfoxid, Dimethylformamid, Formamid, Acetamid, Dimethylacetamid, Butyrolacton, 2-Pyrrolidon und N-Methylpyrrolidon.

Der Begriff Wirkstoffformutierung wird hier und im Folgenden synonym mit dem Begriff Formulierung und Zubereitung verwendet, d. h. im Sinne einer Zusammensetzung, die den Wirkstoff in konzentrierter Form enthält und die für die Anwendung gegebenenfalls mit Wasser oder wässrigen Flüssigkeiten auf die gewünschte Anwendungskonzentration verdünnt wird.

Die erfindungsgemäßen Formulierungen, aber auch die durch Verdünnen einer erfindungsgemäßen Formulierung mit Wasser erhaltenen Wirkstoffaufbereitungen zeichnen sich durch eine äußerst hohe Stabilität gegenüber Entmischungen aus. Sie können ohne Entmischung über einen längeren Zeitraum von mehren Monaten auch bei erhöhter Temperatur und/oder bei stark wechselnden Temperaturen gelagert werden. Zudem lassen sich konzentriertere Zubereitungen auch ohne Probleme mit Wasser verdünnen, ohne dass es zu Entmischungsphänomenen, wie Koagulation, Kristallisation, Flokkulation oder Sedimentation, kommt. Außerdem weisen die erfindungsgemäßen Zusammensetzungen (d.h. wässrige Formulierungen sowie wässrige Wirkstoffaufbereitungen) eine hohe Toleranz gegenüber Elektrolyten auf. Zudem ist auf Grund der äußerst feinen Verteilung, entsprechend dem sehr niedrigen apparenten Teilchendurchmesser der Wirkstoffaggregate die Aktivität der Wirkstoffe im Vergleich zu konventionellen Formulierungen erhöht. Ein weiterer Vorteil ist, dass die erfindungsgemäßen Wirkstoffformulierungen auch lösungsmittelarm (Gehalt an flüchtigen Lösungsmitteln < 10 Gew.-%, bezogen auf das Gewicht der Wirkstoffformulierung) oder sogar lösungsmittelfrei (Gehalt an flüchtigen Lösungsmitteln < 1 Gew.-%, bezogen auf das Gewicht der Wirkstoffformulierung) formuliert werden können.

Ein weiterer Vorteil der Kammpolymere ist darin zu sehen, dass mit ihrer Hilfe Wirkstoffe in fester Form formuliert werden können und diese festen oder pastösen Formulierungen sich mit Wasser verdünnen lassen, wobei die Wirkstoffe in den oben genannten Teilchengrößen erhalten werden.

Im Rahmen der vorliegenden Erfindung wird der Ausdruck "C₁-C₂₀-Alkyl" für eine lineare oder verzweigte Alkylgruppe verwendet, die 1 bis 20 C-Atome aufweist, z. B. für C₁-C₄-Alkyl sowie für Pentyl, Hexyl, 1-Methylpentyl, 2-Methylpentyl, Heptyl, Octyl, 1-Methylheptyl, 2-Methylheptyl, 2,4,4-Trimethylpentan-2-yl, 2-Ethylhexyl, 1-Ethylhexyl, Nonyl, Isononyl, Decyl, 1-Methylnonyl, 2-Propylheptyl und dergleichen. C₁-C₄-Alkyl steht für eine lineare oder verzweigte Alkylgruppe, die 1 bis 4 C-Atome aufweist, z. B. für Methyl, Ethyl, n-Propyl, Isopropyl, n-Butyl, 2-Butyl, 2-Methylpropan-1-yl oder tert.-Butyl.

Der Ausdruck "Cycloalkyl" umfasst im Sinne der vorliegenden Erfindung sowohl unsubstituierte als auch substituierte Cycloalkylgruppen, vorzugsweise C₅-C₇-Cycloalkylgruppen, wie Cyclopentyl, Cyclohexyl oder Cycloheptyl. Diese können im Falle einer Substitution, im Allgemeinen 1, 2, 3, 4 oder 5, bevorzugt 1, 2 oder 3 und besonders bevorzugt 1 Substituenten tragen. Vorzugsweise sind diese Substituenten ausgewählt unter Alkyl, Alkoxy und Halogen.

Der Ausdruck "Phenylalkyl" umfasst im Sinne der vorliegenden Erfindung lineare oder verzweigte Alkylgruppen, insbesondere lineare Alkylgruppen, die durch eine gegebenenfalls substituierte Phenylgruppe substituiert sind, wobei diese Phenylgruppe im Falle einer Substitution im Allgemeinen 1, 2, 3, 4 oder 5, vorzugsweise 1, 2 oder 3 und besonders bevorzugt einen Substituenten, bevorzugt ausgewählt unter Alkyl, Alkoxy, Trifluormethyl, Di(C₁-C₄-alkyl)amino, Nitro, Cyano oder Halogen, trägt. Insbesondere ist die Phenylgruppe in Phenylalkyl unsubstituiert.

Der Ausdruck "Phenoxyalkyl" umfasst im Sinne der vorliegenden Erfindung lineare oder verzweigte Alkylgruppen, Insbesondere lineare Alkylgruppen, die durch eine gegebenenfalls substituierte Phenoxygruppe über das O-Atom der Phenoxygruppe substituiert sind, wobei diese Phenoxygruppe im Falle einer Substitution im Allgemeinen 1, 2, 3, 4 oder 5, vorzugsweise 1 oder 2 Substituenten, bevorzugt ausgewählt unter Alkyl, Alkoxy, Carboxyl, Trifluormethyl, Sulfonat (SO₃⁻), Amino, C₁-C₄-Alkylamino, Di(C₁-C₄-alkyl)amino, Nitro, Cyano oder Halogen, trägt. Insbesondere ist die Phenoxygruppe in Phenoxyalkyl unsubstituiert.

Die hier und im Folgenden angegebenen bevorzugten (oder als häufig oder insbesondere angegebenen), besonders bevorzugten und speziellen Bedeutungen bezüglich der Kammpolymere, insbesondere Art und Mengenanteilen von Monomeren im Kammpolymere, deren Molekulargewicht, sowie bezüglich der Formulierungen und Wirkstoffaufbereitungen, insbesondere bezüglich der Art der Formulierung, der Art der Wirkstoffe, deren Konzentration in den erfindungsgemäßen Formulierungen, Teilchengrößen, Mengenverhältnisse von Kammpolymer zu Wirkstoff, Art und Menge von Zusatzstoffen etc. sind grundsätzlich unabhängig voneinander, bevorzugt jedoch in beliebigen Kombinationen (d.h. in Kombination von zwei oder mehr oder aller dieser Bedeutungen) zu verstehen. D. H. Kombinationen von zwei oder mehr oder aller dieser Bedeutungen stellen bevorzugte, besonders bevorzugter und spezieller Ausführungsformen der Erfindung dar.

Zu den Estern monoethylenisch ungesättigter Monocarbonsäuren mit 3 bis 8 C-Atomen zählen beispielsweise die Ester der Acrylsäure, Methacrylsäure, Crotonsäure und Isocrotonsäure. Zu den Diestern monoethylenisch ungesättigten Dicarbonsäuren mit 4 bis 8 C-Atomen zählen beispielsweise die Diester der Maleinsäure, Fumarsäure und Itaconsäure.

Bevorzugt sind Ester monoethylenisch ungesättigter C₃-C₈-Monocarbonsäuren und hierunter insbesondere die Ester der Acrylsäure und der Methacrylsäure.

Zu den Diestern monoethylenisch ungesättigter C₄-C₈-Dicarbonsäuren zählen insbesondere die Ester der Fumarsäure, der Itaconsäure und der Maleinsäure.

Bevorzugt sind die Monomere Ma ausgewählt unter Estern der Acrylsäure mit C₁-C₂₀-Alkanolen, insbesondere C₁-C₁₀-Alkanolen, wie Methylacrylat, Ethylacrylat, n-Butylacrylat, 2-Butylacrylat, Isobutylacrylat, tert.-Butylacrylat, 2-Ethylhexylacrylat, Decylacrylat, Laurylacrylat und Stearylacrylat, Estern der Acrylsäure mit C₅-C₁₀-Cycloalkanolen wie Cyclohexylacrylat, Estern der Acrylsäure mit Phenyl-C₁-C₄-alkanolen wie Benzylacrylat, 2-Phenylethylacrylat und 1-Phenylethylacrylat, Estern der Acrylsäure mit Phenoxy-C₁-C₄-alkanolen wie 2-Phenoxyethylacrylat, den Estern der Methacrylsäure mit C₁-C₂₀-Alkanolen, vorzugsweise C₁-C₁₀-Alkanolen, wie Methylmethacrylat, Ethylmethacrylat, n-Butylmethacrylat, 2-Butylmethacrylat, Isobutylmethacrylat, tert.-Butylmethacrylat, 2-Ethylhexylmethacrylat, Decylmethacrylat, Laurylmethacrylat und Stearylmethacrylat, Estern der Methacrylsäure mit C₅-C₁₀-Cycloalkanolen wie Cyclohexylmethacrylat, Estern der Methacrylsäure mit Phenyl-C₁-C₄-alkanolen wie Benzylmethacrylat, 2-Phenylethylmethacrylat und 1-Phenylethylmethacrylat, und Estern der Methacrylsäure mit Phenoxy-C₁-C₄-alkanolen wie 2-Phenoxyethylmethacrylat.

In einer besonders bevorzugten Ausführungsform umfassen die Monomere Ma zu wenigstens 80 %, bezogen auf die Gesamtmenge der Monomere Ma, und insbesondere ausschließlich Ester der Acrylsäure und/oder der Methacrylsäure mit C₁-C₁₂-Alkanolen. In einer ganz besonders bevorzugten Ausführungsform umfassen die Monomere Ma zu wenigstens 80 %, bezogen auf die Gesamtmenge der Monomere Ma, und insbesondere ausschließlich Ester der Acrylsäure und/oder der Methacrylsäure mit C₁-C₆-Alkanolen, speziell Methylacrylat, Methylmethacrylat, und/oder Butylacrylat. In einer ebenfalls ganz besonders bevorzugten Ausführungsform umfassen die Monomere Ma zu wenigstens 80 %, bezogen auf die Gesamtmenge der Monomere Ma, und insbesondere ausschließlich ein Gemisch wenigstens eines Monomers Ma(1), das ausgewählt ist unter Estern der Acrylsäure und der Methacrylsäure mit C₁-C₅-Alkanolen, speziell Methylacrylat, Methylmethacrylat, und/oder Butylacrylat, mit wenigstens einem Monomer Ma(2), das ausgewählt ist unter Estern der Acrylsäure und der Methacrylsäure mit C₆-C₂₀-Alkanolen, speziell Laurylacrylat, Stearlyacrylat, Laurylmethacrylat, und/oder Stearylmethacrylat, und Estern der Acrylsäure und der Methacrylsäure mit Phenoxy-C₁-C₄-alkanolen wie 2-Phenoxyethylacrylat und 2-Phenoxyethylmethacrylat.

Vorzugsweise beträgt der Anteil an Monomeren Ma, bezogen auf die Gesamtmonomerenmenge der Monomere M, 10 bis 90 Gew.-%, bevorzugt 20 bis 88 Gew.-% und speziell 40 bis 85 Gew.-%.

Die monoethylenisch ungesättigen Monomere Mb sind ausgewählt unter Polyethergruppenhaltigen Estern, wobei diese aus einer monoethylenisch ungesättigten C₃-C₈-Mono- oder C₄-C₈-Dicarbonsäure sowie einem respektive zwei Poly-(C₂-C₄)-alkylenetherolen aufgebaut sind.

Die den Monomeren Mb zugrunde liegenden Polyalkylenetherole sind linear oder verzweigt, insbesondere linear. In Abhängigkeit des Verzweigungsgrades und der Endgruppen kann es sich bei den den Estern zugrunde liegenden Poly-(C₂-C₄)-alkylenetherolen sowohl um Monoole als auch Polyole, z. B. Diole oder Triole, handeln. Bevorzugt sind Monoole und Diole, insbesondere Monoole.

Es hat sich als vorteilhaft erwiesen, wenn wenigstens 50 mol-%, insbesondere wenigstens 80 mol-%, besonders bevorzugt 90 mol-% und insbesondere alle der die Poly(C₂-C₄-alkylenether)ole bildenden Wiederholungseinheiten für CH₂-CH₂-O stehen. D.h., in den Poly(C₂-C₄-alkylenether)olen beträgt der Anteil einpolymerisierten Ethylenoxides wenigstens 50 mol-%, insbesondere wenigstens 80 mol-%, besonders bevorzugt 90 mol-% und insbesondere 100 mol-%, bezogen auf die Gesamtmenge aller Wiederholungseinheiten im Poly(C₂-C₄-alkylenether)ol. Bei diesen %-Angaben handelt es sich um das Zahlenmittel, bezogen auf die Gesamtmenge an Polyethergruppen im Kammpolymer.

Bevorzugt sind die den Monomeren Mb zugrunde liegenden Poly(C₂-C₄-alkylenether)ole ausgewählt unter Poly-C₂-C₄-alkylenglykol-mono-C₁-C₁₀-alkylethern. Insbesondere weisen die den Monomeren Mb zugrunde liegenden Poly(C₂-C₄-alkylenether)ole ein zahlenmittleres Molekulargewicht im Bereich von 200 bis 10000, speziell im Bereich von 800 bis 2000 auf.

Bevorzugt sind die den Monomeren Mb zugrunde liegenden Poly-(C₂-C₄)-alkylenetherole ausgewählt unter Alkoholen der allgemeinen Formel (I)

HO-(Alk-O)ₙ-Z (I)

worin
- Z: für Wasserstoff, C₁-C₂₀-Alkyl oder Benzyl, insbesondere für C₁-C₁₀-Alkyl, speziell für Methyl oder Ethyl steht,
- n: für eine ganze Zahl steht, deren Zahlenmittel im Bereich von 5 bis 300, vorzugs- weise von 5 bis 200, insbesondere von 10 bis 100 und speziell von 15 bis 50 liegt,
- Alk: für C₂-C₄-Alkylen, insbesondere 1,2-Ethandiyl und/oder 1,2-Propandiyl steht, wo- bei Alk in jeder Wiederholungseinheit (Alk-O)ₙ jeweils gleich oder verschieden sein kann.

Insbesondere sind monoethylenisch ungesättigte Monomere Mb bevorzugt, die unter Estern der allgemeinen Formel (II) ausgewählt sind

A-B-CH=C(R¹)-C(=O)-O-(Alk-O)ₙ-Z (II),

worin
- Z: für Wasserstoff, C₁-C₂₀-Alkyl oder Benzyl, insbesondere für C₁-C₁₀-Alkyl steht,
- B: für eine chemische Bindung oder CH₂, insbesondere für eine chemische Bindung steht,
- n: für eine ganze Zahl steht, deren Mittelwert, bezogen auf das Kammpolymer im Bereich von 5 bis 300, vorzugsweise von 5 bis 200, insbesondere von 10 bis 100 und speziell von 15 bis 50 liegt,
- Alk: für C₂-C₄-Alkylen, insbesondere 1,2-Ethandiyl und/oder 1,2-Propandiyl steht, wo- bei Alk in jeder unterschiedlichen Wiederholungseinheit (Alk-O)ₙ jeweils gleich oder verschieden sein kann,
- R¹: für Wasserstoff oder Methyl steht, insbesondere für Methyl, und
- A: für Wasserstoff, C(O)OH oder C(O)O(Alk'-O)ₘZ' steht, worin Alk' eine der zuvor für Alk gegebenen Bedeutungen besitzt,

Z' eine der zuvor für Z gegebenen Bedeutungen besitzt und
m eine der zuvor für n gegebenen Bedeutungen besitzt,
wobei A insbesondere für Wasserstoff steht.

Im Hinblick auf die erfindungsgemäße Verwendung hat es sich als vorteilhaft erwiesen, wenn die mittlere Anzahl an Wiederholungseinheiten Alk-O in den Gruppen (Alk-O)ₙ bzw. (Alk'-O)ₘ, d. h. das Zahlenmittel von n bzw. m in Formeln (I) und (II) wenigstens 5, insbesondere wenigstens 10 und speziell wenigstens 15 beträgt und einen Wert von 200, insbesondere 100 und speziell 50 nicht überschreitet. Vorzugsweise liegt der Wert im Bereich von 5 bis 200, insbesondere im Bereich von 10 bis 100 und speziell im Bereich von 15 bis 50. Bei dem Mittelwert von n bzw. m handelt es sich um das Zahlenmittel, bezogen auf alle Polyethergruppen im Kammpolymer.

In den Gruppen (Alk-O)ₙ bzw. (Alk'-O)ₘ können die Alkylenteile der einzelnen Wiederholungseinheiten Alk-O bzw. Alk'-O gleich oder verschieden sein. Insbesondere bevorzugt steht Alk-O bzw. Alk'-O für 1,2-Ethandiyl oder Mischungen von 1,2-Ethandiyl mit 1,2-Propandiyl. Sofern die Gruppen (Alk-O)ₙ bzw. (Alk'-O)ₘ voneinander verschiedene Einheiten Alk-O bzw. Alk'-O aufweisen, können diese statistisch oder blockweise angeordnet sein, wobei eine blockweise Anordnung bevorzugt ist.

Sofern die Gruppen (Alk-O)ₙ bzw. (Alk'-O)ₘ verschiedene Wiederholungseinheiten Alk-O bzw. Alk'-O aufweisen, hat es sich als vorteilhaft erwiesen, wenn im Mittel wenigstens 50 mol-%, z. B. 50 bis 99 mol-%, insbesondere wenigstens 80 mol-%, z. B. 80 bis 99 mol-%, und speziell wenigstens 90 mol-%, z. B. 90 bis 98 mol-% der Gruppen Alk-O bzw. Alk'-O für CH₂-CH₂-O stehen. Hierunter sind solche Mischungen bevorzugt, in denen die verbleibenden Wiederholungseinheiten Alk-O bzw. Alk'-O für CH(CH₃)-CH₂-O stehen.

Insbesondere sind die Monomeren Mb ausgewählt unter Estern monoethylenisch ungesättigter C₃-C₈-Monocarbonsäuren mit Poly-(C₂-C₄-alkylenether)olen. Insbesondere sind die Monomere Mb ausgewählt ist unter Estern der Acrylsäure und der Methacrylsäure mit Poly-(C₂-C₄-alkylenether)olen. Insbesondere sind die Monomere Mb unter den Estern der Acrylsäure und der Methacrylsäure mit den zuvor als bevorzugt angegebenen Poly-(C₂-C₄-alkylenether)olen und speziell mit den als bevorzugt angegebenen Poly-(C₂-C₄-alkylenether)monoolen ausgewählt. Ganz besonders bevorzugt sind die Monomere Mb unter den Estern der Acrylsäure und der Methacrylsäure mit den zuvor als bevorzugt angegebenen Poly-C₂-C₄-alkylenglykol-mono-C₁-C₁₀-alkylethem ausgewählt. In diesen bevorzugten und ganz besonders bevorzugten Estern der Acrylsäure und der Methacrylsäure gilt selbstredend das zuvor für das Molekulargewicht der Polyetherole und die Art und Anzahl der Wiederholungseinheiten Gesagte.

Der Anteil der Monomeren Mb, bezogen auf die Gesamtmonomerenmenge der Monomere M liegt typischerweise im Bereich von 10 bis 90 Gew.-%, bevorzugt 12 bis 80 Gew.-% und speziell 15 bis 60 Gew.-%.

Neben den Monomeren Ma und Mb können die das Kammpolymere konstituierenden Monomere M noch weitere, von den Monomeren Ma und Mb verschiedene Monomere Mc umfassen. Der Anteil der Monomere Mc an der Gesamtmenge der Monomere M macht vorzugsweise nicht mehr als 40 Gew.-%, häufig nicht mehr als 30 Gew.-% und insbesondere nicht mehr als 20 Gew.-% aus.

In einer bevorzugten Ausführungsform umfassen die Monomere 5 Gew.-% bis 40 Gew.-%, insbesondere 5 Gew.-% bis 30 Gew.-% und speziell 5 Gew.-% bis 20 Gew.-% der von den Monomeren Ma und Mb verschiedene Monomere Mc.

Die Monomere Mc sind vorzugsweise monoethylenisch ungesättigt, d. h. sie weisen nur eine polymerisierbare, ethylenisch ungesättigte Doppelbindung auf.

Die Monomere Mc umfassen sowohl ionische oder ionisierbare (d.h. saure oder basische) Monomere Mc^{a} als auch neutrale Monomere Mc^{b}. Ionisch bedeutet, dass die Monomere Mc^{a} eine funktionelle Gruppe aufweisen, die in geladenem Zustand vorliegt. Beispiele für ionische Gruppen sind anionische Gruppen wie Carboxylat, Sulfonat, Phosphonat und Phosphat und kationische Gruppen, insbesondere quartäre Ammoniumgruppen und protonierte Aminogruppen, einschließlich quartärer und protonierter Imidazolium-Gruppen und Pyridiniumgruppen. Ionisierbar bedeutet, dass die Monomere Mc^{a} eine funktionelle Gruppe aufweisen, die (im Falle einer basischen, ionisierbaren Gruppe) durch Protonierung oder (im Falle einer sauren, ionisierbaren Gruppe) durch Deprotonierung in eine ionische Gruppe, wie zuvor genannt, überführt werden kann.

Bevorzugte Monomere Mc^{a} sind unter anionischen und sauren Monomere Mc^{a} ausgewählt, insbesondere unter solchen, die wenigstens eine Carbonsäuregruppe oder eine Sulfonsäuregruppe aufweisen.

Zu den bevorzugten Monomeren Mc^{a} zählen insbesondere monoethylenisch ungesättigte Monomere mit wenigstens einer Carbonsäuregruppe, insbesondere monoethylenisch ungesättigte Mono- und Dicarbonsäuren mit 3 bis 8 C-Atomen, wie Acrylsäure, Methacrylsäure, Vinylessigsäure, Crotonsäure, Fumarsäure, Maleinsäure, Itaconsäure, und dergleichen, sowie die Anhydride der vorgenannten monoethylenisch ungesättigten Dicarbonsäuren, wobei der Anteil der Monomere Mc in der Regel 20 Gew.-% und insbesondere 10 Gew.-%, bezogen auf die Gesamtmonomermenge M nicht überschreitet.

Zu den Monomeren Mc^{a} zählen weiterhin monoethylenisch ungesättigte Monomere, die wenigstens eine Sulfonsäuregruppe aufweisen. Beispiele für solche Monomere Mc^{a} sind Styrolsulfonsäure, Vinylsulfonsäure, Allylsulfonsäure, Methallylsulfonsäure sowie die durch die folgende allgemeine Formel III definierten Monomere.

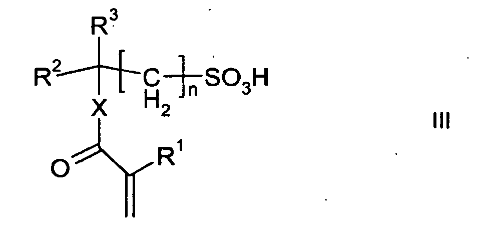

In Formel III bedeuten:
- n: 0, 1, 2 oder 3, insbesondere 1 oder 2;
- X: O oder NR⁵;
- R¹: Wasserstoff oder Methyl;
- R², R³: unabhängig voneinander Wasserstoff oder C₁-C₄-Alkyl, insbesondere Wasserstoff oder Methyl und
- R⁵: Wasserstoff oder C₁-C₄-Alkyl, insbesondere Wasserstoff.

Beispiele für Monomere Mc^{a} der allgemeinen Formel I sind 2-Acrylamido-2-methylpropansulfonsäure, 2-Methacrylamido-2-methylpropansulfonsäure, 2-Acrylamidoethansulfonsäure, 2-Methacrylamidoethansulfonsäure, 2-Acryloyloxyethansulfonsäure, 2-Methacryloyloxyethansulfonsäure, 3-Acryloyloxypropansulfonsäure und 2-Methacryloyloxypropansulfonsäure. In bevorzugten Ausführungsformen enthalten die Kammpolymere keine Monomere mit Sulfonsäuregruppen einpolymerisiert.

Zu den Monomeren Mc^{b} zählen neutrale monoethylenisch ungesättigte Monomere, die im Unterschied zu den Monomeren Mc^{a} keine ionische oder ionisierbare Gruppe aufweisen. Hierunter bevorzugt sind insbesondere solche Monomere Mc^{b}, die eine erhöhte Wasserlöslichkeit aufweisen, insbesondere eine Löslichkeit von wenigstens 60 g/l bei 25°C und 1013 mbar. Solche Monomere mit erhöhter Wasserlöslichkeit oder gar Wassermischbarkeit sind dem Fachmann bekannt, z.B. aus Ullmann's Encyclopedia of Industrial Chemistry, "Polyacrylates", 5th ed. on CD-ROM, Wiley-VCH, Weinheim 1997. Typische Monomere Mc^{b} sind Hydroxy-C₂-C₄-alkylester monoethylenisch ungesättigter Monocarbonsäuren, insbesondere der Acrylsäure und der Methacrylsäure wie 2-Hydroxyethylacrylat, 2-Hydroxypropylacrylat, 3-Hydroxypropylacrylat, 2-Hydroxybutylacrylat, 4-Hydroxybutylacrylat, 2-Hydroxyethylmethacrylat, 2-Hydroxypropylmethacrylat, 3-Hydroxypropylmethacrylat, 2-Hydroxybutylmethacrylat, 4-Hydroxybutylmethacrylat, weiterhin Amide monoethylenisch ungesättigter Monocarbonsäuren wie Acrylamid, Methacrylamid, weiterhin Acrylnitril und Methacrylnitril, N-Vinyllactame wie N-Vinylpyrrolidon, N-Vinylcaprolactam, N-Vinylamide aliphatischer C₁-C₄-Monocarbonsäuren wie N-Vinylformamid, N-Vinylacetamid, monoethylenisch ungesättigte, Harnstoffgruppen tragende Monomere wie N-Vinyl- und N-Allylharnstoff sowie Derivate des Imidazolidin-2-ons, z.B. N-Vinyl- und N-Allylimidazolidin-2-on, N-Vinyloxyethylimidazolidin-2-on, N-Allyloxyethylimidazolidin-2-on, N-(2-Acrylamidoethyl)imidazolidin-2-on, N-(2-Acryloxyethyl)imidazolidin-2-on, N-(2-Methacrylamidoethyl)imidazolidin-2-on, N-(2-Methacryloxyethyl)imidazolidin-2-on (= Ureidomethacrylat), N-[2-(Acryloxyacetamido)ethyl]imidazolidin-2-on, N-[2-(2-Acryloxyacetamido)-ethyl]imidazolidin-2-on, N-[2-(2-Methacryloxyacetamido)ethyl]imidazolidin-2-on; und dergleichen. Vorzugsweise sind die Monomere Mc^{b} ausgewählt unter Hydroxy-C₁-C₄-alkylestern der Acrylsäure und der Methacrylsäure, Acrylamid, Methacrylamid, Acrylnitril, N-Vinyllactamen, wobei die Hydroxy-C₂-C₄-alkylester der Acrylsäure und der Methacrylsäure besonders bevorzugt sind. In einer ganz besonders bevorzugten Ausführungsform umfassen die Monomere Mc^{b} zu wenigstens 80 Gew.-%, bezogen auf die Gesamtmenge der Monomere Mc, wenigstens einen Hydroxy-C₂-C₄-alkylester der Acrylsäure und/oder der Methacrylsäure und/oder N-Vinyl-Caprolactam.

In einer bevorzugten Ausführungsform sind die Monomere Mc ausgewählt unter den Monomeren Mc^{a}, insbesondere unter Monomeren Mc^{a} mit einer Wasserlöslichkeit oberhalb 60 g/l bei 20°C und speziell unter monoethylenisch ungesättigten Monomeren mit wenigstens einer Carbonsäuregruppe. In einer speziellen Ausführungsform sind die Monomere Mc ausgewählt unter monoethylenisch ungesättigten C₃-C₈-Monocarbonsäuren und monoethylenisch ungesättigten C₄-C₈-Dicarbonsäuren und insbesondere unter monoethylenisch ungesättigten C₃-C₆-Monocarbonsäuren und monoethylenisch ungesättigten C₄-C₆-Dicarbonsäuren

In einer ebenfalls bevorzugten Ausführungsform sind die Monomere Mc ausgewählt unter den Monomeren Mc^{b}, insbesondere unter Monomeren Mc^{b} mit einer Wasserlöslichkeit oberhalb 60 g/l bei 20°C und speziell unter Amiden der Acrylsäure und der Methacrylsäure, den Hydroxy-C₁-C₄-alkylestern der Acrylsäure und der Methacrylsäure, und N-Vinyllactamen, wobei Hydroxy-C₂-C₄-alkylester der Acrylsäure und/oder der Methacrylsäure und N-Vinyl-Caprolactam besonders bevorzugt sind.

Erfindungsgemäß bevorzugt sind weiterhin Kammpolymere, die ein zahlenmittleres Molekulargewicht Mₙ im Bereich von 2000 bis 500000 Dalton, häufig im Bereich von 5000 bis 100000 Dalton und insbesondere 10000 bis 50000 Dalton aufweisen. Das gewichtsmittlere Molekulargewicht liegt in der Regel im Bereich von 2000 bis 1000000 Dalton, häufig im Bereich von 3000 bis 200000 Dalton, insbesondere 4000 bis 100000 Dalton und speziell 10000 bis 50000 Dalton. Das Verhältnis M_{w}/Mₙ liegt häufig im Bereich von 1,1:1 bis 10:1, insbesondere im Bereich von 1,2:1 bis 5:1. Die Molmassen M_{w} und Mₙ sowie die Uneinheitlichkeit der Kammpolymere werden durch Größenausschlusschromatographie (= Gelpermeationschromatographie oder kurz GPC) bestimmt. Als Kalibrationsmaterial können handelsübliche Polymethylmethacrylat (PMMA)-Eichsätze verwendet werden.

In der Regel wird das Kammpolymer eine Glasübergangstemperatur T_{g} im Bereich von -80 bis 160°C und häufig im Bereich von -40°C bis +100°C aufweisen. Unter der Glasübergangstemperatur T_{g} wird hier die gemäß ASTM D 3418-82 durch Differentialthermoanalyse (DSC) ermittelte "midpoint temperature" verstanden (vgl. Ullmann's Encyclopedia of Industrial Chemistry, 5th Edition, Volume A 21, VCH Weinheim 1992, S. 169 sowie Zosel, Farbe und Lack 82 (1976), S. 125-134, siehe auch DIN 53765).

In diesem Zusammenhang erweist es sich als hilfreich, die Glasübergangstemperatur T_{g} des Kammpolymers mit Hilfe der Gleichung von Fox (T.G. Fox, Bull. Am. Phys. Soc. (Ser. II) 1, 123 [1956] und Ullmanns Enzyklopädie der technischen Chemie, Weinheim (1980), S. 17-18) anhand der Glasübergangstemperatur der jeweiligen Homopolymere der das Polymer konstituierenden Monomere M abzuschätzen. Letztere sind z. B. aus Ullmann's Encyclopedia of Industrial Chemistry, VCH, Weinheim, Vol. A 21 (1992) S. 169 oder aus J. Brandrup, E.H. Immergut, Polymer Handbook 3rd ed., J. Wiley, New York 1989 bekannt.

Ein Verfahren zur Herstellung der Kammpolymere, umfasst die radikalische Polymerisation der Monomere M.

Die in den erfindungsgemäßen Zubereitungen enthaltenen Kammpolymere können nach üblichen Methoden durch radikalische Polymerisation der Monomere M hergestellt werden. Die Polymerisation kann durch freie radikalische Polymerisation oder durch kontrollierte radikalische Polymerisationsverfahren erfolgen. Die Polymerisation kann unter Einsatz eines oder mehrerer Initiatoren und als Lösungspolymerisation, als Emulsionspolymerisation, als Suspensionspolymerisation oder als Fällungspolymerisation oder auch in Substanz durchgeführt werden. Die Polymerisation kann als Batchreaktion, in semikontinuierlicher oder kontinuierlicher Fahrweise durchgeführt werden.

Die Reaktionszeiten liegen im Allgemeinen im Bereich zwischen 1 und 12 Stunden. Der Temperaturbereich, in dem die Reaktionen durchgeführt werden können, reicht im Allgemeinen von 20 bis 200 °C, bevorzugt von 40 bis 120°C. Der Polymerisationsdruck ist von untergeordneter Bedeutung und kann im Bereich von Normaldruck oder leichtem Unterdruck, z. B. > 800 mbar oder bei Überdruck, z. B. bis 10 bar erfolgen, wobei höhere oder niedrigere Drücke ebenfalls angewendet werden können.

Als Initiatoren für die radikalische Polymerisation werden übliche radikalbildende Substanzen eingesetzt. Bevorzugt sind Initiatoren aus den Gruppen der Azoverbindungen, der Peroxidverbindungen und der Hydroperoxidverbindungen ausgewählt. Zu den Peroxidverbindungen zählen beispielsweise Acetylperoxid, Benzoylperoxid, Lauroylperoxid, tert-Butylperoxy-isobutyrat, Caproylperoxid. Zu den Hydroperoxiden zählen neben Wasserstoffperoxid auch organische Peroxide wie Cumolhydroperoxid, tert.-Butylhydroperoxid, tert.-Amylhydroperoxid und dergleichen. Zu den Azoverbindungen zählen beispielsweise 2,2'-Azobis-isobutyronitril, 2,2'-Azobis(2-methylbutyronitril), 2,2'-Azobis[2-methyl-N-(2-hydroxyethyl)propionamid], 1,1'-Azobis(1-cyclohexancarbonitril), 2,2'-Azobis(2,4-dimethylvaleronitril), 2,2'-Azobis(N,N'-dimethylenisobutyroamidin). Besonders bevorzugt ist Azobisisobutyronitril (AIBN). Üblicherweise setzt man den Initiator in einer Menge von 0,02 bis 5 Gew.-% und insbesondere 0,05 bis 3 Gew.-%, bezogen auf die Menge der Monomere M ein, wobei man auch größere Mengen einsetzen kann, z. B. bis zu 30 Gew.-%, beispielsweise im Falle des Wasserstoffperoxids. Die optimale Menge an Initiator hängt naturgemäß von dem eingesetzten Initiatorsystem ab und kann vom Fachmann in Routineexperimenten ermittelt werden.

Der Initiator kann teilweise oder vollständig im Reaktionsgefäß vorgelegt werden. Vorzugsweise gibt man die Hauptmenge des Initiators, insbesondere wenigstens 80 %, z. B. 80 bis 100 % des Initiators im Verlauf der Polymerisation in den Polymerisationsreaktor.

Selbstverständlich kann das Molekulargewicht der Kammpolymere durch Zugabe von Reglern in einer geringen Menge, z. B. 0,01 bis 5 Gew.-%, bezogen auf die polymerisierenden Monomere M, eingestellt werden. Als Regler kommen insbesondere organische Thioverbindungen z. B. Mercaptoalkohole wie Mercaptoethanol, Mercaptocarbonsäuren wie Thioglykolsäure, Mercaptopropionsäure, Alkylmercaptane wie Dodecylmercaptan, ferner Allylalkohole und Aldehyde in Betracht.

Insbesondere erfolgt die Herstellung der Kammpolymere durch radikalische Lösungspolymerisation in einem organischen Lösungsmittel oder Lösungsmittelgemisch. Beispiele für organische Lösungsmittel sind Alkohole, wie z. B. Methanol, Ethanol, n-Propanol und Isopropanol, dipolar-aprotische Lösungsmittel, z. B. N-Alkyllactame wie N-Methylpyrrolidon (NMP), N-Ethylpyrrolidon, weiterhin Dimethylsulfoxid (DMSO), N,N-Dialkylamide aliphatischer Carbonsäuren wie N,N-Dimethylformamid (DMF), N,N-Dimethylacetamid, weiterhin aromatische, aliphatische und cycloaliphatische Kohlenwasserstoffe, die halogeniert sein können wie Hexan, Chlorbenzol, Toluol oder Benzol sowie Mischungen hiervon. Bevorzugte Lösungsmittel sind Isopropanol, Methanol, Toluol, DMF, NMP, DMSO und Hexan, besonders bevorzugt ist DMF. Weiterhin kann die Herstellung der Kammpolymere in einem Gemisch der zuvor beschriebenen Lösungsmittel und Lösungsmittelgemische mit Wasser erfolgen. Der Wasseranteil dieser Gemische ist dabei bevorzugt kleiner 50 Vol.-% und insbesondere kleiner 10 Vol.-%.

In den erfindungsgemäßen Wirkstoffformulierungen hat es sich als vorteilhaft erwiesen, wenn das Gewichtsverhältnis von Wirkstoff zu Kammpolymer im Bereich von 1 : 10 bis 3 : 1 und insbesondere im Bereich von 1 : 5 bis 2: 1 liegt. Dementsprechend liegt das Kammpolymer in den erfindungsgemäßen Wirkstoffformulierungen typischerweise in einer Menge von 0,3 bis 10 Gew.-Teilen, insbesondere 0,5 bis 8 Gew.-Teilen und speziell 1 bis 5 Gew.-Teilen bezogen auf 1 Gew.-Teil Wirkstoff or.

Eine bevorzugte Ausführungsform der Erfindung betrifft eine feste oder pastöse Wirkstoffformulierung, die wenigstens einen Wirkstoff, der in Wasser bei 25 °C/1013 mbar eine Löslichkeit unterhalb 10 g/l aufweist, und wenigstens ein Kammpolymer enthält. Die Formulierung enthält im Wesentlichen keine oder nur geringe Mengen, d. h. < 10 Gew.-% an Wasser oder organischen Lösungsmitteln. Als weitere Bestandteile können diese Zusammensetzungen die für den jeweiligen Anwendungszweck typischen Hilfsmittel und Additive enthalten. Der Anteil der Hilfsmittel und Additive wird typischerweise 30 Gew.-%, insbesondere 20 Gew.-% und speziell 10 Gew.-%, bezogen auf das Gesamtgewicht der festen bzw. pastösen Wirkstoffformulierung nicht überschreiten. Pastös bedeutet in diesem Zusammenhang, dass die Mischung hochviskos, d. h. zähflüssig ist und zumindest eine honigartige Konsistenz aufweist, wobei die Viskosität typischerweise wenigstens 50 Pa.s, insbesondere wenigstens 100 Pa.s (nach DIN 53019-2 bei 25 °C) beträgt.

Der Anteil an Kammpolymer in der festen oder pastösen Wirkstoffformulierung beträgt typischerweise wenigstens 20 Gew.-% und liegt häufig im Bereich von 20 bis 95 Gew.-%, insbesondere im Bereich von 30 bis 90 Gew.-% und speziell im Bereich von 40 bis 85 Gew.-%, bezogen auf das Gesamtgewicht der Formulierung. Der Anteil an Wirkstoff liegt häufig im Bereich von 5 bis 70 Gew.-%, insbesondere im Bereich von 10 bis 60 Gew.-% und speziell im Bereich von 15 bis 50 Gew.-%. Der Anteil sonstiger Bestandteile wie Wasser, organische Lösungsmittel, Hilfsmittel und Additive wird typischerweise 30 Gew.-%, insbesondere 20 Gew.-% und speziell 10 Gew.-%, bezogen auf das Gesamtgewicht der festen bzw. pastösen Wirkstoffformulierung nicht überschreiten.

Eine weitere Ausführungsform erfindungsgemäßer Formulierungen betrifft eine flüssige Formulierung. Diese flüssigen Formulierungen enthalten wenigstens einen, in Wasser schlecht oder unlöslichen Wirkstoff, wenigstens ein erfindungsgemäßes Kammpolymer und ein flüssiges Lösungs- oder Verdünnungsmittel.

Geeignete Lösungs- bzw. Verdünnungsmittel sind neben Wasser auch organische Lösungsmittel, insbesondere solche, die mit Wasser mischbar sind, sowie Mischungen dieser Lösungsmittel mit Wasser. Beispiele für mit Wasser mischbare Lösungsmittel umfassen C₃-C₄-Ketone wie Aceton und Methylethylketon, cyclische Ether wie Dioxan und Tetrahydrofuran, C₁-C₄-Alkanole wie Methanol, Ethanol, n-Propanol, Isopropanol, n-Butanol, tert.-Butanol, Polyole und deren Mono- und Dimethylether wie Glykol, Propandiol, Ethylenglykolmonomethylether, Diethylenglykol, Diethylenglykolmonomethylether, Diethylenglykoldimethylether, Glycerin, cyclische Carbonate mit 3 bis 6 C-Atomen wie Ethylencarbonat (= 1,3-Dioxolan-2-on), sowie weiterhin C₂-C₃-Nitrile wie Acetonitril und Propionitril, Dimethylsulfoxid, Dimethylformamid, Formamid, Acetamid, Dimethylacetamid, Butyrolacton, 2-Pyrrolidon und N-Methylpyrroildon.

Bevorzugte Verdünnungsmittel ist Wasser oder ein wässriges Medium, das bis 10 Gew.-%, bezogen auf das Gewicht des wässrigen Mediums, eines oder mehrere organische, mit Wasser mischbare Lösungsmittel enthält. Formulierungen, die als Verdünnungsmittel Wasser oder ein wässriges Medium enthalten, umfassen Wasser oder ein wässriges Medium als kontinuierliche Phase und wenigstens eine disperse Phase, die im Wesentlichen aus dem wenigstens einen Wirkstoff und dem wenigstens einen erfindungsgemäßen Kammpolymer bestehen. In diesen wässrigen Wirkstoffformulierungen liegen der Wirkstoff und das Kammpolymer vermutlich in Form von Aggregaten aus Wirkstoff und den erfindungsgemäßen Kammpolymeren vor. Diese wirkstoff-haltige Phase bildet somit eine disperse Phase, die den Wirkstoff und wenigstens ein erfindungsgemäßes Kammpolymer enthält.

Der Wirkstoff liegt in der kontinuierlichen wässrigen Phase in äußerst feinteiliger Form vor. Man nimmt an, dass der Wirkstoff in der wässrigen Phase mit dem erfindungsgemäßen Kammpolymer Aggregate bildet. Diese Aggregate weisen in der Regel mittlere Teilchengrößen unterhalb 1 µm, häufig unterhalb 500 nm, insbesondere unterhalb 400 nm, speziell unterhalb 300 nm und ganz speziell unterhalb 200 nm auf.

Der Anteil an Kammpolymer in der flüssigen Wirkstoffformulierung beträgt typischerweise wenigstens 5 Gew.-% und liegt häufig im Bereich von 5 bis 50 Gew.-%, insbesondere im Bereich von 7 bis 40 Gew.-% und speziell im Bereich von 10 bis 35 Gew.-%, bezogen auf das Gesamtgewicht der Formulierung. Der Anteil an Wirkstoff liegt häufig im Bereich von 0,5 bis 40 Gew.-%, insbesondere im Bereich von 1 bis 30 Gew.-% und insbesondere im Bereich von 5 bis 25 Gew.-%. Der Anteil an Verdünnungsmittel liegt typischerweise im Bereich von 10 bis 94,5 Gew.-%, insbesondere im Bereich von 25 bis 92 Gew.-% und speziell im Bereich von 30 bis 85 Gew.-%. Der Anteil sonstiger Hilfsmittel und Additive wird typischerweise 10 Gew.-%, insbesondere 5, bezogen auf das Gesamtgewicht der flüssigen Wirkstoffformulierung nicht überschreiten.

Die Herstellung der erfindungsgemäßen Wirkstoffformulierung kann in unterschiedlicher Weise erfolgen. Typischerweise umfasst die Herstellung der erfindungsgemäßen Wirkstoffformulierung die Herstellung bzw. Bereitstellung einer homogenen, nichtwässrigen Mischung, umfassend die erfindungsgemäße Kammpolymere und wenigstens einen Wirkstoff.

Gemäß einer ersten bevorzugten Ausführungsform der vorliegenden Erfindung umfasst das Verfahren die Herstellung einer homogenen, nicht wässrige Mischung, die wenigstens ein erfindungsgemäßes Kammpolymer und wenigstens einen Wirkstoff enthält. Diese Mischung ist, wenn sie keine flüssigen Bestandteile enthält, in der Regel fest oder hochviskos bzw. pastös und wird hier und im Weiteren als feste oder pastöse Formulierung bezeichnet.

Zum Herstellen der homogenen, nicht wässrigen Mischung wird man in der Regel den Wirkstoff in eine flüssige Form des Kammpolymers, beispielsweise in eine Schmelze des Kammpolymers oder vorzugsweise in eine Lösung des Kammpolymers in einem organischen Lösungsmittel einarbeiten. Das Einarbeiten kann auch durch Extrusion einer Mischung von Wirkstoff und Kammpolymer und gegebenenfalls Lösungsmittel erfolgen. Sofern man ein Lösungsmittel verwendet, wird man anschließend das Lösungsmittel möglichst weitgehend und vorzugsweise vollständig entfernen, z. B. durch Destillation (oder im Falle einer Extrusion z.B. auch mittels eines Entgasungsextruders), wobei man eine feste oder hochviskose, bzw. pastöse Lösung des Wirkstoffs in dem Kammpolymer erhält. Geeignete Lösungsmittel hierfür sind grundsätzlich solche, die sowohl den Wirkstoff als auch das Kammpolymer zu lösen vermögen, beispielsweise aliphatische Nitrile wie Acetonitril und Propionitril, N,N-Dialkylamide aliphatischer Carbonsäuren wie Dimethylformamid und Dimethylacetamid, N-Alkyllactame wie N-Methylpyrrolidon, die vorgenannten aliphatischen und alicyclischen Ether, beispielsweise Tetrahydrofuran, halogenierte Kohlenwasserstoffe wie Dichlormethan, Dichlorethan, Ester aliphatischer C₁-C₄-Carbonsäuren mit C₁-C₆-Alkanolen wie Ethylacetat, Butylacetat, Butylformiat, Methylpropionat, Methybutyrat und Mischungen der vorgenannten Lösungsmittel. An dieser Stelle können gegebenenfalls gewünschte Additive und Hilfsmittel in an sich bekannter Weise in die Zusammensetzung eingearbeitet werden.

Diese Vorgehensweise ist grundsätzlich auch zur Herstellung lösungsmittelhaltiger Formulierungen geeignet, wobei man dann in der Regel auf ein Entfernen des Lösungsmittels verzichten kann oder das zur Herstellung der homogenen Mischung gegebenenfalls eingesetzte Lösungsmittel durch ein anderes organisches Lösungsmittel ersetzt.

Die erfindungsgemäßen festen Wirkstoffformulierungen können auch durch Trocknen einer erfindungsgemäßen flüssigen Formulierung, insbesondere durch Trocknen einer wässrigen Wirkstoffformulierung hergestellt werden. Beispielsweise lassen sich die erfindungsgemäße flüssige Wirkstoffformulierungen, insbesondere wässrigen Wirkstoffformulierungen aber auch Lösungen des Wirk- bzw. Effektstoffs und der Kammpolymere in einem organischen Lösungsmittel zu einem redispergierbaren festen Material wie z. B. Pulver oder Granulate trocknen. D.h. durch Entfernen der wässrigen Phase bzw. des organischen Lösungsmittels während des Trocknens erhält man je nach Trocknungsbedingungen feinteilige Pulver oder grobteilige Granulate, die sich ohne weiteres in Wasser lösen oder dispergieren lassen, ohne dass eine nennenswerte Teilchenvergrößerung eintritt.

Dementsprechend betrifft eine bevorzugte Ausführungsform der Erfindung feste Wirkstoffformulierungen, die durch Trocknen der wässrigen Wirkstoffformulierungen erhalten werden. Hierbei handelt es sich um feste Materialien, die üblicherweise partikelförmig anfallen. Je nach Art des Trocknungsverfahrens erhält man z. B. Pulver oder Granulate.

Zur Trocknung entfernt man die flüchtigen Bestandteile, d. h. Wasser und/oder organische Lösungsmittel, nach üblichen Methoden. Zu nennen sind insbesondere die konvektive Trocknungsmethoden, wie die Sprühtrocknung, Sprühwirbelschichttrocknung, Stromtrocknung, Mahltrocknung, Bandtrocknung und Mischformen dieser Trocknungsmethoden, durch Kontakttrocknungsverfahren wie Walzentrocknung, Kammertrocknung, Dünnschichttrocknung, Trocknung in einem Schaufeltrockner oder in einem Trommeltrockner, Gefriertrocknung, und Strahlungstrocknung. Derartige Verfahren sind dem Fachmann geläufig, z. B. aus C. M. van't Land "Industrial Drying Equipment" Marcel Decker, Inc. 1991; O. Krischer, W. Kast, K. Kröll, "Trocknungstechnik", Bd. 1 bis 3, Springer-Verlag 1978, 1959 und 1989; K. Masters, Spraydrying Handbook Longman Scientific and Technical; H. Uhlmann/Lothar Mörl, Wirbelschicht/Sprühgranulat, Springerverlag 2000. Vorzugsweise erfolgt die Trocknung der wässrigen Wirkstoffformulierungen bei Temperaturen unterhalb des Glaspunktes des Kammpolymers und insbesondere im Bereich von 20 °C bis 100 °C.

In einer zweiten Ausführungsform der vorliegenden Erfindung stellt man eine wässrige Wirkstoffformulierung her, indem man zunächst eine homogene, nicht wässrige Mischung, umfassend ein Kammpolymer und wenigstens einen Wirkstoff herstellt und die so erhaltene Mischung anschließend in Wasser oder einem wässrigen Medium dispergiert, beispielsweise durch Anwendung von Scherkräften, z.B. durch Rühren oder mittels eines Dispergators. Bezüglich der Herstellung der homogenen, nicht wässrigen Mischung gilt das zuvor im Zusammenhang mit der ersten Ausführungsform für die Herstellung einer homogenen, nicht wässrigen Mischung, umfassend Kammpolymer und Wirkstoff. Gesagte in analoger Weise. Das Dispergieren kann sowohl bei Temperaturen im Bereich der Umgebungstemperatur als auch bei erhöhter Temperatur erfolgen, beispielsweise bei einer Temperatur im Bereich von 10 bis 80 °C und insbesondere im Bereich von 20 bis 50 °C.

In einer weiteren Ausführungsform der vorliegenden Erfindung erfolgt die Herstellung der wässrigen Wirkstoffformulierung durch Einarbeiten des Wirkstoffs in eine wässrige Lösung/Dispersion des Kammpolymers. Hierzu geht man in der Regel so vor, dass man das Einarbeiten bei einer Temperatur durchführt, die oberhalb der Schmelztemperatur des Wirkstoffs liegt und vorzugsweise bei einer Temperatur, bei der die Wirkstoffschmelze niedrigviskos ist, d. h. eine Viskosität im Bereich von 1 bis 1000 mPa.s (nach DIN 53019-2 bei 25 °C) aufweist. Vorzugsweise erfolgt das Einarbeiten unter Anwendung von starken Scherkräften, beispielsweise in einem Ultraturrax.

In einer weiteren Ausführungsform der Erfindung erfolgt die Herstellung der wässrigen Wirkstoffformulierung durch ein Verfahren, umfassend die folgenden Schritte a bis c:
a) Herstellung einer Lösung von Wirkstoff und gegebenenfalls Kammpolymer in einem organischen Lösungsmittel, das einen Siedepunkt unter dem von Wasser aufweist und
b) Vermischen der Lösung des Wirkstoffs mit Wasser oder mit einer wässrigen Lösung des Kammpolymers und
c) Entfernen des Lösungsmittels.

Hierbei kann man alternativ so vorgehen, dass die Lösung des Wirkstoffs das Kammpolymer enthält und man diese Lösung mit Wasser vermischt, oder dass die Lösung des Wirkstoffs nur einen Teil des Kammpolymers oder kein Kammpolymer enthält und man diese Lösung mit einer wässrigen Lösung oder Dispersion des Kammpolymers vermischt. Das Vermischen kann in geeigneten Rührgefäßen erfolgen, wobei man entweder Wasser oder die wässrige Lösung des Kammpolymers vorlegen kann und hierzu die Lösung des Wirkstoffs gibt, oder alternativ die Lösung des Wirkstoffs vorlegt und hierzu das Wasser bzw. die wässrige Lösung des Kammpolymers gibt. Anschließend entfernt man das organische Lösungsmittel ganz oder teilweise, z. B. durch Destillation, wobei man gegebenenfalls Wasser zusetzt.

In einer bevorzugten Variante dieser Ausführungsform gibt man die Wirkstofflösung und das Wasser bzw. die wässrige Lösung des Kammpolymers kontinuierlich in eine Mischzone und entnimmt dieser kontinuierlich die Mischung, aus der man anschließend das Lösungsmittel ganz oder teilweise entfernt. Die Mischzone kann beliebig ausgestaltet werden. Grundsätzlich sind hierfür alle Apparaturen geeignet, die ein kontinuierliches Mischen von Flüssigkeitsströmen ermöglichen. Derartige Apparaturen sind bekannt, z. B. aus Continuous Mixing of Fluids (J.-H. Henzler) in Ullmann's Encyclopedia 5th ed. on CD-Rom, Wiley-VCH. Die Mischzonen können als statische oder dynamische Mischer oder Mischformen davon ausgestaltet sein. Als Mischzonen kommen insbesondere auch Jet-Mischer oder vergleichbare Mischer mit Düsen in Betracht. In einer bevorzugten Ausgestaltung handelt es sich bei der Mischzone um die im "Handbook of Industrial Crystallization" (A. S. Myerson, 1993 Butterworth-Heinemann, Seite 139, ISBN 0-7506-9155-7) beschriebene Apparatur oder eine vergleichbare Apparatur.

Das Volumenverhältnis von Wirkstofflösung zu Wasser bzw. wässriger Lösung des Kammpolymers kann über weite Bereich variiert werden und liegt vorzugsweise im Bereich von 10 : 1 bis 1 : 20 und insbesondere im Bereich von 5 : 1 bis 1 : 10.

Naturgemäß sollte das Lösungsmittel geeignet sein, das Kammpolymer und den Wirkstoff in den gewünschten Mengenverhältnissen zu lösen. Geeignete Lösungsmittel kann der Fachmann durch Routineexperimente ermitteln. Beispiele für geeignete Lösungsmittel sind C₂-C₄-Alkanole wie Ethanol, n-Propanol, n-Butanol, Isobutanol, die vorgenannten aliphatischen und alicyclischen Ether wie Diethylether, Diisopropylether, Methyl-tert.-butylether, Dioxan, Tetrahydrofuran, Ketone wie Aceton, Methylethylketon, Lactone wie gamma-Butyrolacton, Carbonate wie Diethylcarbonat, Ethylencarbonat, Propylencarbonat, Lactame wie Pyrrolidon, N-methylpyrrolidon, N-Ethylpyrrolidon, Caprolactam, Amide aliphatischer Carbonsäuren wie Acetamid, N,N-Dimethylacetamid, N,N-Dimethylformamid, Nitrile wie Acetonitril und Propionitril halogenierte Kohlenwasserstoffe wie Dichlormethan, Dichlorethan, Ester aliphatischer C₁-C₄-Carbonsäuren mit C₁-C₆-Alkanolen wie Ethylacetat, Butylacetat, Butylformiat, Methylpropionat, Methybutyrat und dergleichen sowie Mischungen der vorgenannten organischen Lösungsmittel.

Der Gehalt an Wirkstoff kann über weite Bereiche variiert werden. Insbesondere ermöglichen die Kammpolymere das Herstellen von so genannten Wirkstoffkonzentraten, welche den Wirkstoff in einer Menge von wenigstens 5 Gew.-%, z. B. in einer Menge von 5 bis 50 Gew.-% und insbesondere in einer Menge von 5 bis 40 Gew.-%, bezogen auf das Gesamtgewicht der Formulierung, enthalten.

Vorteilhafterweise können die erfindungsgemäßen Formulierungen, insbesondere die wässrigen Wirkstoffformulierungen und die festen bzw. pastösen Formulierungen lösungsmittelfrei oder lösungsmittelarm formuliert werden, d. h. der Anteil an flüchtigen, organischen Bestandteilen in den Wirkstoffformulierungen beträgt häufig nicht mehr als 10 Gew.-%, insbesondere nicht mehr als 5 Gew.-% und speziell nicht mehr als 1 Gew.-%, bezogen auf das Gesamtgewicht der Formulierung. Flüchtige Bestandteile sind dabei solche, die bei Normaldruck einen Siedepunkt unterhalb 200 °C aufweisen.

In den erfindungsgemäßen Formulierungen können eine Vielzahl unterschiedlicher Wirkstoffe formuliert werden. Insbesondere eignen sich die erfindungsgemäßen Kammpolymere zur Formulierungen organischer Wirkstoffe, insbesondere niedermolekularer Wirkstoffe mit einem Molekulargewicht von nicht mehr als 2000 Dalton, insbesondere nicht mehr 1000 Dalton, z. B. im Bereich von 100 bis 1000 Dalton und speziell im Bereich von 150 bis 500 Dalton. Eine besondere Ausführungsform der Erfindung betrifft die Formulierung von Wirkstoffen für den Pflanzenschutz, d. h. von Herbiziden, Fungiziden, Nematiziden, Akariziden, Insektiziden sowie Wirkstoffen, die das Pflanzenwachstum regulieren.

Insbesondere handelt es sich bei den erfindungsgemäß verwendeten Wirkstoffen um insektizide und/oder fungizide Wirkstoffe.

Beispiele für fungizide Wirkstoffe, die unter Verwendung der erfindungsgemäßen Kammpolymere zu einer Wirkstoffformulierung formuliert werden können, umfassen die folgenden organischen Verbindungen:
- Strobilurine, wie z. B. Azoxystrobin, Dimoxystrobin, Enestroburin, Fluoxastrobin, Kresoxim-methyl, Metominostrobin, Picoxystrobin, Pyraclostrobin, Trifloxystrobin, Orysastrobin, (2-Chlor-5-[1-(3-methyl-benzyloxyimino)-ethyl)-benzyl)-carbaminsäuremethylester, (2-Chlor-5-[1-(6-methyl-pyridin-2-ylmethoxyimino)-ethyl]-benzyl)-carbaminsäuremethylester, 2-(ortho-(2,5-Dimethylphenyloxymethyl)phenyl)-3-methoxy-acrylsäuremethylester;
   Carbonsäureamide
- Carbonsäureanilide, wie z. B. Benalaxyl, Benodanil, Boscalid, Carboxin, Mepronil, Fenfuram, Fenhexamid, Flutolanil, Furametpyr, Metalaxyl, Ofurace, Oxadixyl, Oxycarboxin, Penthiopyrad, Thifluzamide, Tiadinil, 4-Difluormethyl-2-methyl-thiazol-5-carbonsäure-(4'-brom-biphenyl-2-yl)-amid, 4-Difluormethyl-2-methyl-thiazol-5-carbonsäure-(4'-trifluormethyl-biphenyl-2-yl)-amid, 4-Difluormethyl-2-methyl-thiazol-5-carbonsäure-(4'-chlor-3'-fluor-biphenyl-2-yl)-amid, 3-Difluormethyl-1-methyl-pyrazol-4-carbonsäure-(3',4'-dichlor-4-fluor-biphenyl-2-yl)-amid, 3-Difluormethyl-1-methyl-pyrazol-4-carbonsäure-(3',4'-di-chlor-5-fluor-biphenyl-2-yl)-amid, 3,4-Dichlor-isothiazol-5-carbonsäure-(2-cyano-phenyl)-amid. Geeignete Carbonsäureanilide sind weiterhin Benaxalyl-M, Bixafen, Isotianil, Kiralaxyl, Tecloftalam, 2-Amino-4-methylthiazol-5-carbonsäureanilid, 2-Chlor-N-(1,1,3-trimethylindan-4-ylnicotinamid, N-(3',4'-Dichlor-5-fluorbiphenyl-2-yl)-3-diffuormethyl-1-methyl-1H-pyrazol-4-carbonsäureamid, N-(3',4'-Dichlor-5-fluorbiphenyl-2-yl)-3-trifluormethyl-1-methyl-1H-pyrazol-4-carbonsäureamid, 5-Fluor-1,3-dimethyl-1H-pyrazol-4-carbonsäure-[2-(1,3-dimethylbutyl)phenyl]amid, N-(4'-Chlor-3',5-difluorbiphenyl-2-yl)-3-difluormethyl-1-methyl-1H-pyrazol-4-carbonsäureamid, N-(4'-Chlor-3',5-difluorbiphenyl-2-yl)-3-trifluormethyl-1-methyl-1H-pyrazol-4-carbonsäureamid, N-(3',5-Difluor-4'-methylbiphenyl-2-yl)-3-difluormethyl-1-methyl-1H-pyrazol-4-carbonsäureamid, N-(3',5-Difluor-4'-methylbiphenyl-2-yl)-3-trifluormethyl-1-methyl-1H-pyrazol-4-carbonsäureamid, N-(2-Bicyclopropyl-2-ylphenyl)-3-difluormethyl-1 -methyl-1H-pyrazol-4-carbonsäureamid, N-(cis-2-Bicyclopropyl-2-ylphenyl-3-difluormethyl-1-methyl-1H-pyrazol-4-carbonsäureamid und N-(trans-2-Bicyclopropyl-2-ylphenyl)-3-difluormethyl-1-methyl-1H-pyrazol-4-carbonsäureamid;
- Carbonsäuremorpholide, wie z. B. Dimethomorph, Flumorph;
- Benzoesäureamide, wie z. B. Flumetover, Fluopicolide (Picobenzamid), Zoxamide. Geeignet ist auch N-(3-Ethyl-3,5,5-trimethylcyclohexyl)-3-formuylamino-2-hydroxybenazmid;
- Sonstige Carbonsäureamide, wie z. B. Carpropamid, Diclocymet, Mandipropamid, N-(2-(4-[3-(4-Chlor-phenyl)-prop-2-inyloxy]-3-methoxy-phenyl)-ethyl)-2-methylsulfonylamino-3-methyl-butyramid, N-(2-(4-[3-(4-Chlor-phenyl)-prop-2-inyloxy]-3-methoxy-phenyl)-ethyl)-2-ethylsulfonylamino-3-methyl-butyramid. Geeignet ist weiterhin Oxytetracyclin, Silthiofam, N-(6-Methoxypyridin-3-yl)cyclopropancarbonsäureamid;
   Azole
- Triazole, wie z. B. Bitertanol, Bromuconazole, Cyproconazole, Difenoconazole, Diniconazole, Enilconazole, Epoxiconazole, Fenbuconazole, Flusilazole, Fluquinconazole, Flutriafol, Hexaconazol, Imibenconazole, lpconazole, Metconazol, Myclobutanil, Penconazole, Propiconazole, Prothioconazole, Simeconazole, Tebuconazole, Tetraconazole, Triadimenol, Triadimefon, Triticonazole;
- Imidazole, wie z. B. Cyazofamid, Imazalil, Pefurazoate, Prochloraz, Triflumizole;
- Benzimidazole, wie z. B. Benomyl, Carbendazim, Fuberidazole, Thiabendazole; und sonstige, wie Ethaboxam, Etridiazole, Hymexazole;
   Stickstoffhaltige Heterocyclylverbindungen
- Pyridine, wie z. B. Fluazinam, Pyrifenox, 3-[5-(4-Chlor-phenyl)-2,3-dimethyl-isoxazolidin-3-yl]-pyridin;
- Pyrimidine, wie z. B. Bupirimate, Cyprodinil, Ferimzone, Fenarimol, Mepanipyrim, Nuarimol, Pyrimethanil;
- Piperazine, wie Triforine;
- Pyrrole, wie Fludioxonil, Fenpiclonil;
- Morpholine, wie Aldimorph, Dodemorph, Fenpropimorph, Tridemorph;
- Dicarboximide, wie lprodione, Procymidone, Vinclozolin;
- Sonstige, wie Acibenzolar-S-methyl, Anilazin, Captan, Captafol, Dazomet, Diclomezine, Fenoxanil, Folpet, Fenpropidin, Famoxadone, Fenamidone, Octhilinone, Probenazole, Proquinazid, Pyroquilon, Quinoxyfen, Tricyclazole, 6-Aryl-[1,2,4]triazolo[1,5-a]pyrimidinen, beispielsweise Verbindungen der nachfolgend definierten Formel (IV), z. B. 5-Chlor-7-(4-methyl-piperidin-1-yl)-6-(2,4,6-trifluorphenyl)-[1,2,4]triazolo[1,5-a]pyrimidin, 2-Butoxy-6-iodo-3-propyl-chromen-4-on, 3-(3-Brom-6-fluoro-2-methyl-indol-1-sulfonyl)-[1,2,4]triazol-1-sulfonsäuredimethylamid;
   Carbamate und Dithiocarbamate
- Dithiocarbamate, wie Ferbam, Mancozeb, Maneb, Metiram, Metam, Propineb, Thiram, Zineb, Ziram;
- Carbamate, wie Diethofencarb, Flubenthiavalicarb, lprovalicarb, Propamocarb, 3-(4-Chlor-phenyl)-3-(2-isopropoxycarbonylamino-3-methyl-butyrylamino)-propionsäuremethylester, N-(1-(1-(4-cyanophenyl)ethylsulfonyl)-but-2-yl) carbaminsäure-(4-fluorphenyl)ester;
   Sonstige Fungizide
- Guanidine, wie Dodine, iminoctadine, Guazatine;
- Antibiotika, wie Kasugamycin, Polyoxine, Streptomycin, Validamycin A;
- Organometallverbindungen, wie Fentin Salze;
- Schwefelhaltige Heterocyclylverbindungen, wie Isoprothiolane, Dithianon;
- Organophosphorverbindungen, wie Edifenphos, Fosetyl, Fosetylaluminium, lprobenfos, Pyrazophos, Tolclofos-methyl, Phosphorige Säure und ihre Salze;
- Organochlorverbindungen, wie Thiophanat Methyl, Chlorothalonil, Dichlofluanid, Tolylfluanid, Flusulfamide, Phthalide, Hexachlorbenzol, Pencycuron, Quintozene;
- Nitrophenylderivate, wie Binapacryl, Dinocap, Dinobuton;
- Sonstige, wie z. B. Spiroxamine, Cyflufenamid, Cymoxanil, Metrafenon.

Beispiele für herbizide Wirkstoffe, die unter Verwendung der Kammpolymere zu Wirkstoffformulierung formuliert werden können, umfassen:
- 1,3,4-Thiadiazole wie Buthidazole und Cyprazole;
- Amide wie Allidochlor, Benzoylpropethyl, Bromobutide, Chlorthiamid, Dimepiperate, Dimethenamid, Diphenamid, Etobenzanid, Flampropmethyl, Fosamin, lsoxaben, Metazachlor, Monalide, Naptalame, Pronamid, Propanil;
- Aminophosphorsäuren wie Bilanafos, Buminafos, Glufosinateammonium, Glyphosate, Sulfosate;
- Aminotriazole wie Amitrol, Anilide wie Anilofos, Mefenacet;
- Anilide wie Anilofos, Mefenacet;
- Aryloxyalkansäure wie 2,4-D, 2,4-DB, Clomeprop, Dichlorprop, Dichlorprop-P, Fenoprop, Fluroxypyr, MCPA, MCPB, Mecoprop, Mecoprop-P, Napropamide, Napro-panilide, Triclopyr;
- Benzoesäuren wie Chloramben, Dicamba;
- Benzothiadiazinone wie Bentazon;
- Bleacher wie Clomazone, Diflufenican, Fluorochloridone, Flupoxam, Fluridone, Pyrazolate, Sulcotrione;
- Carbamate wie Carbetamid, Chlorbufam, Chlorpropham, Desmedipham, Phenmedipham, Vernolate;
- Chinolinsäuren wie Quinclorac, Quinmerac;
- Dichlorpropionsäuren wie Dalapon;
- Dihydrobenzofurane wie Ethofumesate;
- Dihydrofuran-3-one wie Flurtamone;
- Dinitroaniline wie Benefin, Butralin, Dinitramin, Ethalfluralin, Fluchloralin, Isopropalin, Nitralin, Oryzalin, Pendimethalin, Prodiamine, Profluralin, Trifluralin,
- Dinitrophenole wie Bromofenoxim, Dinoseb, Dinoseb-acetat, Dinoterb, DNOC, Medinoterb-Acetat;
- Diphenylether wie Acifluorfen-sodium, Aclonifen, Bifenox, Chlornitrofen, Difenoxuron, Ethoxyfen, Fluorodifen, Fluoroglycofen-ethyl, Fomesafen, Furyloxyfen, Lactofen, Nitrofen, Nitrofluorfen, Oxyfluorfen;
- Dipyridyle wie Cyperquat, Difenzoquat-methylsulfat, Diquat, Paraquat-dichlorid;
- Imidazole wie Isocarbamid;
- Imidazolinone wie Imazamethapyr, Imazapyr, Imazaquin, Imazamethabenzmethyl, Imazethapyr, Imazapic, lmazamox;
- Oxadiazole wie Methazole, Oxadiargyl, Oxadiazon;
- Oxirane wie Tridiphane;
- Phenole wie Bromoxynil, loxynil;
- Phenoxyphenoxypropionsäureester wie Clodinafop, Cyhalofop-butyl, Diclofopmethyl, Fenoxaprop-ethyl, Fenoxaprop-P-ethyl_{;} Fenthiapropethyl, Fluazifopbutyl, Fluazifop-P-butyl, Haloxyfop-ethoxy-ethyl, Haloxyfop-methyl, Haloxyfop-P-methyl, Isoxapyrifop, Propaquizafop, Quizalofop-ethyl, Quizalofop-P-ethyl, Quizalofop-tefuryl;
- Phenylessigsäuren wie Chlorfenac;
- Phenylpropionsäuren wie Chlorphenprop-methyl;
- ppi-Wirkstoffe wie Benzofenap, Flumiclorac-pentyl, Flumioxazin, Flumipropyn, Flupropacil, Pyrazoxyfen, Sulfentrazone, Thidiazimin;
- Pyrazole wie Nipyraclofen;
- Pyridazine wie Chloridazon, Maleic hydrazide, Norflurazon, Pyridate;
- Pyridincarbonsäuren wie Clopyralid, Dithiopyr, Picloram, Thiazopyr;
- Pyrimidylether wie Pyrithiobacsäure, Pyrithiobac-sodium, KIH-2023, KIH-6127;
- Sulfonamide wie Flumetsulam, Metosulam;
- Triazolcarboxamide wie Triazofenamid;
- Uracile wie Bromacil, Lenacil, Terbacil;
- ferner Benazolin, Benfuresate, Bensulide, Benzofluor, Bentazon, Butamifos, Cafenstrole, Chlorthal-dimethyl, Cinmethylin, Dichlobenil, Endothall, Fluorbentranil, Mefluidide, Perfluidone, Piperophos, Topramezone und Prohexadion-Calcium;
- Sulfonylharnstoffe wie Amidosulfuron, Azimsulfuron, Bensulfuron-methyl, Chlorirnuron-ethyl, Chlorsulfuron, Cinosulfuron, Cyclosulfamuron, Ethametsulfuron-methyl, Flazasulfuron, Halosulfuron-methyl, Imazosulfuron, Metsulfuronmethyl, Nicosulfuron, Primisulfuron, Prosulfuron, Pyrazosulfuron-ethyl, Rimsulfuron, Sulfometuron-methyl, Thifensulfuron-methyl, Triasulfuron, Tribenuronmethyl, Triflusulfuron-methyl, Tritosulfuron;
- Pflanzenschutz-Wirkstoffe vom Cyclohexenon-Typ wie Alloxydim, Clethodim, Cloproxydim, Cycloxydim, Sethoxydim und Tralkoxydim. Ganz besonders bevorzugte herbizide Wirkstoffe vom Cyclohexenon-Typ sind: Tepraloxydim (vgl. AGROW, Nr. 243, 3.11.95, Seite 21, Cycloxydim) und 2-(1-[2-{4-Chlorphenoxy}propyl-oxyimino]butyl)-3-hydroxy-5-(2H-tetrahydrothiopyran-3-yl)-2-cyclohexen-1-on und vom Sulfonylharnstoff Typ: N-(((4-methoxy-6-[trifluormethyl]-1,3,5-triazin-2-yl)amino)carbo-nyl)-2-(trifluormethyl)-benzolsulfonamid.

Beispiele für Insektizide, die unter Verwendung der Kammpolymere zu Wirkstoffformulierung formuliert werden können, umfassen:
- Organo(thio)phosphate wie Acephate, Azamethiphos, Azinphos-ethyl, Azinphos-methyl, Cadudsafos, Chlorethoxyphos, Chlorfenvinphos, Chlormephos, Chlorpyrifos, Chlorpyrifos-methyl, Chlorfenvinphos, Coumaphos, Cyanophos, Demeton-S-methyl, Diazinon, Dichlorvos/ DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isoxathion, Malathion, Mecarbam, Methamidophos, Methidathion, Methyl-parathion, Mevinphos, Monocrotophos, Naled, Omethoate, Oxydemeton-methyl, Paraoxon, Parathion, Parathion-methyl, Phenthoat, Phorat, Phosalon, Phosmet, Phosphamidon, Phorate, Phoxim, Pirimiphos, Pirimiphos-methyl, Profenofos, Propetamphos, Prothiofos, Pyraclofos, Pyridaphenthion, Quinalphos, Sulfotep, Sulprophos, Tebupirimfos, Temephos, Terbufos, Tetrachlorvinphos, Thiometon, Triazophos, Trichlorfon, Vamidothion;
- Carbamate wie Alanycarb, Aldicarb, Bendiocarb, Benfuracarb, Butocarboxim, Butoxycarboxim, Carbaryl, Carbofuran, Carbosulfan, Ethiofencarb, Fenobucarb, Fenoxycarb, Formetanat, Furathiocarb, Isoprocarb, Methiocarb, Methomyl, Metolcarb, Oxamyl, Pirimicarb, Propoxur, Thiodicarb, Thiofanox, Triazemate, Trimethacarb, XMC, Xylylcarb;
- Pyrethroide, wie Acrinathrin, Allethrin, d-cis-trans Allethrin, d-trans Allethrin, Bifenthrin, Bioallethrin, Bioallethrin S-cyclopentenyl, Bioresmethrin, Cycloprothrin, Cyfluthrin, beta-Cyfluthrin, Cyhalothrin, lambda-Cyhalothrin, gamma-Cyhalothrin, Cyphenothrin, Cypermethrin, alpha-Cypermethrin, beta-Cypermethrin, theta-Cypermethrin, zeta-Cypermethrin, Deltamethrin, Empenthrin, Esfenvalerat, Etofenprox, Fenpropathrin, Fenvalerat, Flucythrinat, Flumethrin, tau-Fluvalinate, Halfenprox, Imiprothrin, Permethrin, Phenothrin, Prallethrin, Profluthrin, Pyrethrin I and II, Resmethrin, RU 15525, Silafluofen, tau-Fluvalinate, Tefluthrin, Tetramethrin, Tralomethrin, Transfluthrin, Dimefluthrin, ZXI 8901;
- Arthropode Wachstumsregulatoren: a) Chitinsyntheseinhibitoren z. B. Benzoylharnstoffe wie Bistrifluron, Chlorfluazuron, Diflubenzuron, Flucycloxuron, Flufenoxuron, Hexaflumuron, Lufenuron, Novaluron, Noviflumuron, Teflubenzuron, Triflumuron, Buprofezin, Diofenolan, Hexythiazox, Etoxazole, Clofentezine; b) Ecdysone antagonisten, wie Chromafenozide, Halofenozide, Methoxyfenozide, Tebufenozide, Azadirachtin; c) Juvenoide wie Pyriproxyfen, Hydroprene, Kinoprene, Methoprene, Fenoxycarb; d) Lipid-Biosyntheseinhibitors wie Spirodiclofen, Spiromesifen, Spirotetramat;
- Agonisten/Antagonisten der Nicotin Rezeptoren: Acetamiprid, Clothianidin, Dinotefuran, Imidacloprid, Nitenpyram, Thiacloprid, Thiamethoxam, Nicotin, Bensultap, Cartap-hydrochloride, Thiocyclam, Natrium-Thiosultap; die Thiazolverbindung der Formel (Γ¹) 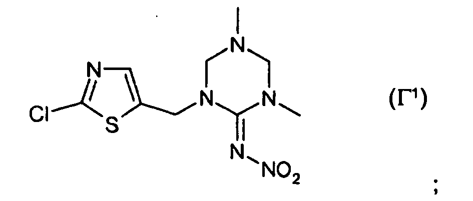
- GABA Antagonisten wie Acetoprol, Chlordan, Endosulfan, Ethiprol, gamma-HCH (Lindan), Fipronil, Vaniliprol, Pyrafluprol, Pyriprol, Vaniliprol, Phenylpyrazol-Verbindungen der Formel Γ² 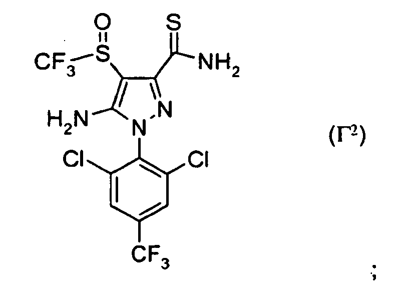
- Macrocyclische Lactone wie Abamectin, Emamectin, Emamectinbenzoate, Milbemectin, Lepimectin, Spinosad;
- METI I Verbindungen wie Fenazaquin, Fenpyroximat, Flufenerim, Pyridaben, Pyrimidifen, Rotenon, Tebufenpyrad, Tolfenpyrad;
- METI II und III Verbindungen wie Acequinocyl, Fluacrypyrim, Hydramethylnon;
- Entkopplungsverbindungen wie Chlorfenapyr, DNOC;
- Inhibitoren der oxidativen Phosphorylierung wie Azocyclotin, Cyhexatin, Diafenthiuron, Fenbutatinoxid, Propargit, Tetradifon;
- Verschiedene Oxidaseinhibitoren wie Piperonylbutoxid;
- Natrium-Kanal-Blocker wie Indoxacarb, Metaflumizone;
- Microbielle Disruptoren wie Bacillus thuringiensis subsp. israelensis, Bacillus sphaericus, Bacillus thuringiensis subsp. aizawai, Bacillus thuringiensis subsp. kurstaki, Bacillus thuringiensis subsp. tenebrionis;
- Weitere wie Amitraz, Benclothiaz, Benzoximat, Bifenazat, Bromopropylat, Cartap, Chinomethionat, Chloropicrin, Flonicamid, Methylbromid, Pyridalyl, Pymetrozin, Rynaxypursulfur, Brechweinstein, Thiocyclam, Tribufosflubendiamide, Cyenopyrafen, Flupyrazofos, Cyflumetofen, Amidoflumet, NNI-0101, N-R'-2,2-Dihalo-1-R"-cyclopropancarboxamid-2-(2,6-dichlor- α,α.α-trifluor-p-tolyl)hydrazon oder N-R'-2,2-Di(R''')propionamid-2-(2,6-dichloro-α,α,α-trifluoro-p-tolyl)-hydrazon, worin R' für Methyl oder Ethyl steht, Halo für Chlor oder Brom steht, R" für Wasserstoff oder Methyl steht und R''' für Methyl oder Ethyl steht, Anthranilamide der Formel Γ³ 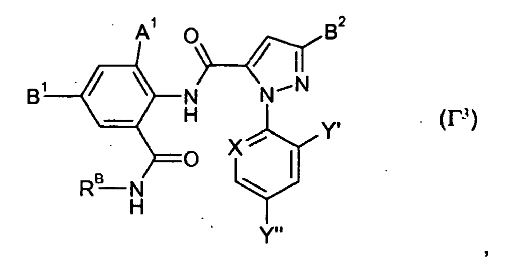 worin A¹ für CH₃, Cl, Br, I steht, X für C-H, C-Cl, C-F oder N steht, Y' für F, Cl oder Br steht, Y" für F, Cl, CF₃ steht, B¹ für Wasserstoff, Cl, Br, I, CN steht, B² für Cl, Br, CF₃, OCH₂CF₃, OCF₂H steht and R^{B} für Wasserstoff, CH₃ oder CH(CH₃)₂ steht, und Malononitrile wie in JP 2002 284608, WO 02/89579, WO 02/90320, WO 02/90321, WO 04/06677, WO 04/20399 oder JP 2004 99597 beschrieben;
- Malodinitrile wie CF₃(CH₂)₂C(CN)₂CH₂(CF₂₎₃CF₂H, CF₃(CH₂)₂C(CN)₂CH₂(CF₂)₅CF₂H, CF₃(CH₂)₂C(CN)₂(CH₂)₂C(CF₃)₂F, CF₃(CH₂)₂C(CN)₂(CH₂)₂(CF₂)₃CF₃, CF₂H(CF₂)₃CH₂C(CN)₂CH₂(CF₂)₃CF₂H, CF₃(CH₂)₂C(CN)₂CH₂(CF₂)₃CF₃, CF₃(CF₂)₂CH₂C(CN)₂CH₂(CF₂)₃CF₂H, CF₃CF₂CH₂C(CN)₂CH₂(CF₂)₃CF₂H, 2-(2,2,3,3,4,4,5,5-Octafluorpentylr2-(3,3,4,4,4-pentafluorbutyl)-malonodinitril und
- CF₂HCF₂CF₂CF₂CH₂C(CN)₂CH₂CH₂CF₂CF₃
- Fluorierte Quinazolinone wie: 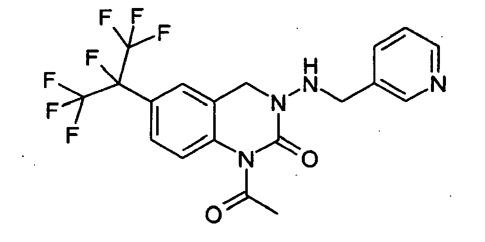 1-Acetyl-3-[(pyridin-3-ylmethyl)-amino]-6-(1,2,2,2-tetrafluoro-1-trifluoromethylethyl)-3,4-dihydro-1H-quinazolin-2-on;
- Außerdem Pyrimidinylalkinylether der Formel Γ⁴ oder Thiadiazolylalkinylether der Formel Γ⁵: 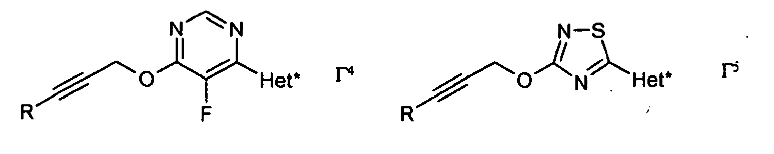 worin R für Methyl oder Ethyl steht und Het* für 3,3-Dimethylpyrrolidin-1-yl, 3-Methylpiperidin-1-yl, 3,5-Dimethylpiperidin-1-yl, 4-Methylpiperidin-1-yl, Hexahydroazepin-1-yl, 2,6-Dimethylhexahydroazepin-1-yl oder 2,6-Dimethylmorpholin-4-yl. Diese Verbindungen werden beispielsweise in JP 2006 131529 beschrieben.

Eine bevorzugte Ausführungsform der Erfindung betrifft die Verwendung der erfindungsgemäßen Kammpolymere zur Herstellung von Wirkstoffformulierungen von in Wasser unlöslichen oder schlecht löslichen Fungiziden bzw. die Verwendung der erfindungsgemäßen Kammpolymere zur Solubilisierung von in Wasser unlöslichen oder schlecht löslichen Fungiziden in einem wässrigen Medium.

In einer bevorzugten Ausführungsform ist der formulierte Wirkstoff ausgewählt unter
- Strobilurinen, z. B. Azoxystrobin, Dimoxystrobin, Fluoxastrobin, Kresoxim-methyl, Metominostrobin, Orysastrobin, Picoxystrobin, Pyraclostrobin sowie Trifloxystrobin, insbesondere Pyraclostrobin,
- Conazolfungiziden, insbesondere Prochloraz, Cyproconazol, Epoxyconazol, Fluquinconazol, Hexaconazol, Metconazol, Penconazol, Propiconazol, Prothioconazol, Tebuconazol und Triticonazol und speziell Epoxyconazol, Metconazol, Fluquinconazol oder Prothioconazol,
- 6-Aryl-[1,2,4]triazolo-[1,5-a]-pyrimidinen, insbesondere unter Wirkstoffen der allgemeinen Formel (IV) wie nachstehend definiert und
- Wirkstoffen der allgemeinen Formel IV 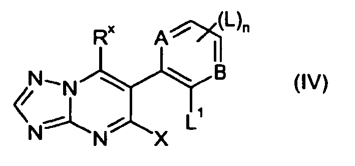
- Mischungen dieser Wirkstoffe.

In Formel IV haben die Variablen die folgenden Bedeutungen:
- R^{x}: ist ausgewählt unter NR¹⁴R¹⁵, gegebenenfalls substituiertem C₁-C₈-Alkyl, C₂-C₆-Alkenyl, C₃-C₆-Cycloalkyl, C₃-C₆-Cycloalkenyl, Phenyl oder Naphthyl, wo- bei die 4 zuletzt genannten Reste gegebenenfalls substituiert sein können;
R¹⁴, R¹⁵ sind unabhängig voneinander ausgewählt unter Wasserstoff, C₁-C₈-Alkyl, C₁-C₈-Halogenalkyl, C₃-C₁₀-Cydoalkyl, C₃-C₆-Halogencycloalkyl, C₂-C₈-Alkenyl, C₄-C₁₀-Alkadienyl, C₂C₈-Halogenalkenyl, . C₃-C₈,-Cyvlloalkenyl, C₂-C₈-Halogencydoalkenyl, C₂-C₈-Alkinyl, C₂-C₈-Halogenalkinyl oder C₃-C₆-Cycloalkinyl; oder
R¹⁴ zusammen mit R¹⁵ und dem Stickstoffatom, an das sie gebunden sind, ste- hen für fünf- bis achtgliedriges Heterocyclyl, welches über N gebunden ist und gegebenenfalls 1, 2 oder 3 weitere Heteroatome aus der Gruppe O, N und S als Ringglied enthält und gegebenenfalls substituiert ist;
- n: steht für 1, 2, 3 oder 4;
- A: CH, C-L oder N;
- B: CH, C-L oder N;
- L: ist bzw. sind unabhängig voneinander ausgewählt unter Halogen, Cyano, C₁-C₆- Alkyl, C₁-C₄-Halgoenalkyl, C₁-C₆-Alkoxy, C₁-C₄-Halgoenalkoxy und C₁-C₆- Alkoxycarbonyl;
- L¹: ist ausgewählt ist unter Wasserstoff, Halogen, C₁-C₆-Alkyl und C₁-C₆- Halogenalkyl;
- X: ist ausgewählt ist unter Halogen, C₁-C₄-Alkyl, Cyano, C₁-C₄-Alkoxy oder C₁-C₄-Halogenalkyl.

Besonders bevorzugte Beispiele für Verbindungen der Formel IV sind 5-Chlor-7-(4-methylpiperidin-1-yl)-6-(2,4,6-trifluorphenyl)-[1,2,4]triazolo[1,5-a]pyrimidin, 6-(3,4-Dichlor-phenyl)-5-methyl-[1,2,4]triazolo[1,5-a]pyrimidin-7-ylamin, 6-(4-tert-Butylphenyl)-5-methyl-[1,2,4]triazolo[1,5-a]pyrimidin-7-ylamin, 5-Methyl-6-(3,5,5-trimethylhexyl)-[1,2,4]triazoto[1,5-a]pyrimidin-7-ylamin, 5-Methyl-6-octyl-[1,2,4]triazolo[1,5-a]pyrimidin-7-ylamin, 5-Ethyl-6-octyl-[1,2,4]triazolo[1,5-a]pyrimidin-2,7-diamin, 6-Ethyl-5-octyl-[1,2,4]triazolo[1,5-a]pyrimidin-7-ylamin, 5-Ethyl-6-octyl-[1,2,4]triazolo[1,5-a]pyrimidin-7-ylamin, 5-Ethyl-6-(3,5,5-trimethyl-hexyl)-[1,2,4]triazolo[1,5-a]pyrimidin-7-ylamin, 6-Octyl-5-propyl-[1,2,4]triazolo[1,5-a]pyrimidin-7-ylamin, 5-Methoxymethyl-6-octyl-[1,2,4]triazolo[1,5-a]pyrimidin-7-ylamin, 6-Octyl-5-trifluormethyl-[1,2,4]triazolo[1,5-a]pyrimidin-7-ylamin, 5-Trifluormethyl-6-(3,5,5-trimethyl-hexyl)-[1,2,4]triazolo[1,5-a]pyrimidin-7-ylamin und deren landwirtschaftlich geeignten Salze.

In einer weiteren Ausführungsform der erfindungsgemäßen Wirkstoffformulierungen umfassen diese eine Kombination aus wenigstens zwei Wirkstoffen, insbesondere wenigstens zwei Fungizide. Speziell handelt es sich bei der Wirkstoffkombination um eine Kombination von wenigstens einemconazolfungizid, speziell Epoxiconazol mit wenigstens einem Strobilurin, insbesondere Pyraclostrobin, und gegebenenfalls einem weiteren Wirkstoff, z. B. Fenpropidin; um eine Kombination von zwei verschiedenen Conazolfungiziden, speziell Epoxiconazol mit wenigstens einem weiteren, von Epoxiconazol verschiedenen Conazolfungizid, insbesondere mit einem Conazolfungizid, das unter Prochloraz, Cyproconazol, Fluquinconazol, Hexaconazol, Metconazol, Penconazol, Propiconazol, Prothioconazol, Tebuconazol und Triticonazol und speziell Metconazol, Fluquinconazol und Prothioconazol ausgewählt ist; sowie um eine Kombination von wenigstens einem 6-Aryl-[1,2,4]triazolo-[1,5-a]-pyrimidinen, insbesondere einem Wirkstoff der allgemeinen Formel (IV), speziell 5-Chlor-7-(4-methylpiperidin-1-yl)-6-(2,4,6-trifluorphenyl)-[1,2,4]triazolo[1,5-a]pyrimidin mit wenigstens einem anderen fungiziden Wirkstoffen, speziell mit einem oder mehreren Conazolfungiziden.

Eine weitere bevorzugte Ausführungsform der Erfindung betrifft die Verwendung der Kammpolymere zur Herstellung von Wirkstoffformulierungen von Insektiziden, insbesondere von Arylpyrrolen wie Chlorfenapyr, von Pyrethroiden wie Bifenthrin, Cyfluthrin, Cycloprothrin, Cypermethrin, Deltamethrin, Esfenvalerate, Ethofenprox, Fenpropathrin, Fenvalerate, Cyhalothrin, Lambda-Cyhalothrin, Permethrin, Silafluofen, Tau-Fluvalinate, Tefluthrin, Tralomethrin, alpha-Cypermethrin, Zeta-Cypermethrin und Permethrin, von Neonicotinoiden und von Semicarbazonen wie Metaflumizone.

Eine bevorzugte Ausführungsform der Erfindung betrifft demnach auch die Verwendung der Kammpolymere zur Stabilisierung bzw. Solubilisierung von Insektiziden, insbesondere von Arylpyrrolen, von Pyrethroiden, von Neonicotinoiden und von Metaflumizone, in wässriger Phase.

Außerdem werden die Kammpolymere zur Herstellung von Wirkstoffformulierungen, insbesondere wässriger Wirkstoffformulierungen von pharmazeutischen Wirkstoffen und Pro-Drug eingesetzt. Hierzu zählen Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, lmmunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, lmmuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika. Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel. Beispiele für geeignete pharmazeutische Wirkstoffe sind die insbesondere die in den Absätzen 0105 bis 0131 der US 2003/0157170 genannten Wirkstoffe.

Weiterhin eignen sich die Kammpolymere zur Herstellung von Zubereitungen, insbesondere wässriger Zubereitungen von kosmetischen Wirkstoffen, insbesondere von kosmetischen Öle und Fetten wie Erdnussöl, Jojobaöl, Kokosnussöl, Mandelöl, Olivenöl, Palmöl, Ricinusöl, Sojaöl oder Weizenkeimöl, ätherische Öle wie Latschenkiefernöl, Lavendelöl, Rosmarinöl, Fichtennadelöl, Kiefernnadelöl, Eukalyptusöl, Pfefferminzöl, Salbeiöl, Bergamottöl, Terpentinöl, Melissenöl, Wacholderöl, Zitronenöl, Anisöl, Kardamonöl, Campheröl etc. oder für Mischungen aus diesen Ölen.

Außerdem eignen sich die Kammpolymere zur Herstellung von Zubereitungen, insbesondere wässriger Zubereitungen von Nahrungsergänzungsmitteln wie wasserunlöslichen Vitaminen und Provitaminen wie Vitamin A, Vitamin A-Acetat, Vitamin D, Vitamin E, Tocopherol-Derivate wie Tocopherolacetat, Coenzym Q10 und Vitamin K.

Dementsprechend eignen sich die Kammpolymere auch zur Stabilisierung der vorgenannten Wirkstoffe in wässriger Phase.

Die erfindungsgemäßen Zusammensetzungen können in fester Form oder in flüssiger Form formuliert sein. Je nach Ausgestaltung können die erfindungsgemäßen Zusammensetzungen noch Hilfsmittel und/oder Träger enthalten, wie sie in Pflanzenschutzmitteln oder in Mitteln für den Materialschutz üblich sind. Zu den Hilfsmitteln zählen insbesondere konventionelle oberflächenaktive Substanzen und sonstige im Pflanzen- und Materialschutz übliche Additive und Trägerstoffe, die fest oder flüssig sein können. Zu den oberflächenaktiven Substanzen zählen insbesondere Tenside, insbesondere solche, die Netzmitteleigenschaften haben. Zu den sonstigen Hilfsmitteln (Additive) zählen insbesondere Verdickungsmittel, Entschäumer, Konservierungsmittel, Frostschutzmittel, Stabilisierungsmittel, Anticaking-Mittel bzw. Rieselhilfsmittel und Puffer.

Prinzipiell brauchbare konventionelle oberflächenaktive Substanzen sind anionische, nichtionische und amphotere Tenside, einschließlich Polymer-Tenside, wobei das Molekulargewicht der Tenside typischerweise einen Wert von 2000 Dalton und insbesondere 1000 Dalton nicht überschreiten wird (Zahlenmittel)..

Zu den anionischen Tensiden gehören beispielsweise Carboxylate, insbesondere Alkali-, Erdalkali- und Ammoniumsalze von Fettsäuren, z. B. Kaliumstearat, die üblicherweise auch als Seifen bezeichnet werden; Acylglutamate; Sarkosinate, z. B. Natriumlauroylsarkosinat; Taurate; Methylcellulosen; Alkylphosphate, insbesondere Mono- und Diphosphorsäurealkylester; Sulfate, insbesondere Alkylsulfate und Alkylethersulfate; Sulfonate, weitere Alkyl- und Alkylarylsulfonate, insbesondere Alkali-, Erdalkali- und Ammoniumsalze von Arylsulfonsäuren sowie alkylsubstituierten Arylsulfonsäuren, Alkylbenzolsulfonsäuren, wie beispielsweise Lignin- und Phenolsulfonsäure, Naphthalin- und Dibutylnaphthalinsulfonsäuren, oder Dodecylbenzolsulfonate, Alkylnaphthalinsulfonate, Alkylmethylestersulfonate, Kondensationsprodukte von sulfoniertem Naphthalin und Derivaten davon mit Formaldehyd, Kondensationsprodukte von Naphthalinsulfonsäuren, Phenol- und/oder Phenolsulfonsäuren mit Formaldehyd oder mit Formaldehyl und Harnstoff, Mono- oder Dialkylbernsteinsäureestersulfonate; sowie Eiweißhydrolysate und Lignin-Sulfitablaugen. Die zuvor genannten Sulfonsäuren werden vorteilhafterweise in Form ihrer neutralen oder gegebenenfalls basischen Salze verwendet.

Zu den nichtionischen Tensiden gehören beispielsweise:
- Fettalkoholalkoxylate und Oxoalkoholalkoxylate, insbesondere Ethoxylate und Propoxylate mit Alkoxilierungsgraden von üblicherweise 2 bis 100 und insbesondere 3 bis 50, z. B. Alkoxylate von C₈-C₃₀-Alkanolen oder Alk(adi)enolen, z. B. von iso-Tridecylalkohol, Laurylalkohol, Oleylalkohol oder Stearylalkohol sowie deren C₁-C₄-Alkylether und C₁-C₄-Alkylester z.B. deren Acetate;
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Zuckertenside, Sorbitester, wie beispielsweise Sorbitanfettsäureester (Sorbitanmonooleat, Sorbitantristearat), Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Zu den amphoteren Tensiden gehören beispielsweise Sulfobetaine, Carboxybetaine und Alkyldimethylaminoxide, z. B. Tetradecyldimethylaminoxid.

Weitere Tenside, die hier beispielhaft genannt werden sollen, sind Perfluortenside, Silikontenside, Phospholipide, wie beispielsweise Lecithin oder chemisch modifizierte Lecithine, Aminosäuretenside, z. B. N-Lauroylglutamat.

Sofern nicht spezifiziert, handelt es sich bei den Alkylketten der oben aufgeführten Tenside um lineare oder verzweigte Reste mit üblicherweise 6 bis 30 und insbesondere 8 bis 20 Kohlenstoffatomen.

In einer Ausführungsform enthalten die erfindungsgemäßen wässrigen Zusammensetzungen nicht mehr als 10 Gew.-%, vorzugsweise nicht mehr als 5 Gew.-% und insbesondere nicht mehr als 3 Gew.-%, z. B. 0,01 bis 5 Gew.-% oder 0,1 bis 3 Gew.-% an konventionellen oberflächenaktiven Substanzen, jeweils bezogen auf die Gesamtmenge an Wirkstoff und Kammpolymer.

Je nach Anwendung kann es jedoch von Vorteil sein, wenn die erfindungsgemäßen Wirkstoffformulierungen mit oberflächenaktiven Substanzen formuliert werden. Dann liegt der Anteil an konventioneller oberflächenaktiver Substanz häufig im Bereich von 0,1 bis 60 Gew.-%, insbesondere im Bereich von 0,5 bis 50 Gew.-%, bezogen auf die Gesamtmenge an Wirkstoff und Kammpolymer, bzw. im Bereich von 0,1 bis 60 Gew.-%, insbesondere im Bereich von 0,5 bis 50 Gew.-% und speziell im Bereich von 0,5 bis 30 Gew.-%, bezogen auf das Gesamtgewicht der formulierten Zubereitung.

Auch wenn ein Vorteil der erfindungsgemäßen Zusammensetzungen ihr geringer Gehalt an flüchtigen organischen Substanzen ist, kann es für einige Anwendungen erwünscht sein, die erfindungsgemäßen Zusammensetzungen mit organischen Lösungsmitteln, Ölen und Fetten, vorzugsweise solchen Lösungsmitteln oder Ölen und Fetten, die umweltverträglich oder biokompatibel sind, z. B. die vorgenannten mit Wasser mischbaren Lösungsmittel oder Lösungsmittel, Ölen oder Fetten die mit Wasser nicht oder nur sehr begrenzt mischbar sind, abzumischen. Hierzu zählen z. B.:
- Paraffinöle, aromatische Kohlenwasserstoffe und aromatische Kohlenwasserstoffgemische, z. B Xylole, Solvesso 100, 150 oder 200, und dergleichen,
- Phenole und Alkylphenole, z. B. Phenol, Hydrochinon, Nonylphenol, etc.
- Ketone mit mehr als 4 C-Atomen wie Cyclohexanon, Isophoron, Isopheron, Acetophenon, Acetonaphthon,
- Alkohole mit mehr als 4 C-Atomen wie acetylierter Lanolinalkohol, Cetylalkohol, 1-Decanol, 1-Heptanol, 1-Hexanol, Isooctadecanol, lsopropylalkohol, Oleylalkohol, Benzylalkohol,
- Carbonsäureester, z. B. Adipinsäuredialkylester wie Adipinsäure-bis(2-ethylhexyl)ester, Phthalsäuredialkylester wie Phthalsäure-bis(2-ethylhexyl)ester, Essigsäurealkylester (auch verzweigte Alkylgruppen) wie • Ethylacetat und Acetoessigsäureethylester, Stearate wie Butylstearat, Glycerinmonostearat, Citrate wie Acetyltributylcitrat, weiterhin Cetyloctanoat, Methyloleat, Methyl-p-hydroxybenzoat, Methyltetradecanoat, Propyl-p-hydroxybenzoat, Methylbenzoat, Milchsäureester wie Isopropyllacttat, Butyllactat und 2-Ethylhexyllactat,
- Pflanzenöle wie Palmöl, Rapsöl, Rizinusöl und Derivate davon wie z. B. oxydiert, Kokusnussöl, Lebertran, Maiskeimöl, Sojabohnenöl, Leinsamenöl, Olivenöl, Erdnussöl, Färberdistelöl, Sesamsamenöl, Grapefruitöl, Basilikumöl, Aprikosenöl, Ingweröl, Geranienöl, Orangenöl, Rosmarienöl, Macadamiaöl, Zwiebelöl, Mandarinenöl, Kiefernöl, Sonnenblumenöl,
- hydrogenierte Pflanzenöle wie hydrogeniertes Palmöl, hydrogeniertes Rapsöl, hydrogeniertes Sojabohnenöl,
- tierische Öle wie Schweinefettöl, Fischöle,
- Dialkylamide mittel bis langkettiger Fettsäuren z. B. Hallcomide sowie
- Pflanzenölester wie Rapsölmethylester.

Geeignete Verdicker sind Verbindungen, die wässrigen Zusammensetzungen ein pseudoplastisches Fließverhalten verleihen, d. h. hohe Viskosität im Ruhezustand und niedrige Viskosität im bewegten Zustand. Hier sind beispielsweise Polysaccharide wie Xanthan (Kelzan^{®} der Fa. Kelco; Rhodopol^{®} 23 von Rhone Poulenc; oder Veegum^{®} der Firma R.T. Vanderbilt) sowie anorganische Schichtmineralien wie Attaclay^{®} (Firma Engelhardt) zu nennen, wobei Xanthan bevorzugt verwendet wird.

Als für die erfindungsgemäßen Dispersionen geeignete Antischaummittel kommen beispielsweise Silikonemulsionen (wie z. B. Silicon^{®} SRE, Firma Wacker oder Rhodorsil^{®} der Firma Rhodia), langkettige Alkohole, Fettsäuren, fluororganische Verbindungen und deren Gemische in Betracht.

Bakterizide können zur Stabilisierung den erfindungsgemäßen Zusammensetzungen gegen Befall mit Mikroorganismen zugesetzt werden. Hierbei handelt es sich typischerweise um Isothiazolon-Verbindungen, z. B. 1,2-Benzisothiazolin-3-on, 5-Chlor-2-methylisothiazol-3-on, 2-Methylisothiazol-3-on oder 2-Octylisothiazol-3-on, die beispielsweise unter den Handelsbezeichnungen Probel^{®} der Fa. Arch Chemical Inc., Acticide^{e} RS der Fa. Thor Chemie und Kathon^{®} MK der Firma Rohm & Haas erhältlich sind.

Geeignete Frostschutzmittel sind organische Polyole, z. B. Ethylenglycol, Propylenglycol oder Glycerin. Diese werden in wässrigen 1Formulierungen eingesetzt, üblicherweise in Mengen von nicht mehr als 20 Gew.-%, z. B. 1 bis 20 Gew.-% und insbesondere 2 bis 10 Gew.-%, bezogen auf das Gesamtgewicht der wässrigen Wirkstoffzubereitung.

Gegebenenfalls können die erfindungsgemäßen Wirkstoffformulierungen 1 bis 5 Gew.-%, bezogen auf die Gesamtmenge der hergestellten Zubereitung, Puffer zur pH-Wert Regulation der Zubereitung oder der verdünnten Applikationsform enthalten, wobei sich die Menge und Art des eingesetzten Puffers nach den chemischen Eigenschaften und der Menge der Wirkstoffe und des Kammpolymers richtet. Beispiele für Puffer sind Alkalisalze schwacher anorganischer oder organischer Säuren wie z. B. Phosphorsäure, Borsäure, Essigsäure, Propionsäure, Citronensäure, Fumarsäure, Weinsäure, Oxalsäure und Bernsteinsäure.

Beispiele für Rieselhilfsmittel sind insbesondere Kieselsäure, speziell pyrogene Kieselsäure und Fällungskieselsäure sowie Calciumcarbonat und Magnesiumstearat. Die Menge an Rieselhilfsmittel beträgt, sofern vorhanden, typischerweise bis 5 Gew.-%, insbesondere bis 2 Gew.-%, z.B. 0,1 bis 5 Gew.-% oder 0,2 bis 2 Gew.-%, bezogen auf das Gesamtgewicht der Zusammensetzung.

Als Trägerstoffe kommen grundsätzlich alle flüssigen und festen Substanzen in Betracht, die üblicherweise in Formulierungen für den Pflanzenschutz oder Materialschutz, insbesondere in Formulierungen von Fungiziden zum Einsatz kommen und die typischerweise chemisch inert sind. Flüssige Trägerstoffe sind insbesondere Wasser sowie Mischungen von Wasser mit organischen, mit Wasser mischbaren Lösungsmitteln. Feste Trägerstoffe sind z. B. Silikate und Alumosilikate einschließlich Bolus, Löß, Tone und Tonerden, z. B. Phyllosilikate und Tektosilikate wie Montmorillonit, Hektorit, Saponit, Beidellit, Sauconit, Bentonit, Talk, Kaolin, Attapulgit, weiterhin amorphe Silikate und Kieselsäuren wie Kieselgele, Kieselgur, z. B. in Form von Diatomeenerde, Fällungskieselsäure, künstliche Silikate und Alumosilikate wie Zeolithe, weiterhin Kalkstein, Kalk, Kreide, Dolomit, Calcium- und Magnesiumsulfat, Magnesiumoxid, gemahlene Kunststoffe, Düngemittel, wie z. 8. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe. Vorzugsweise sind die festen Trägerstoffe in Wasser löslich oder dispergierbar.

Je nach Art des enthaltenen Wirkstoffs können die erfindungsgemäβen Wirkstoffformulierungen in an sich mitkonventionellen Formulierungen des jeweiligen Wirkstoffs vergleichbarer Weise eingesetzt werden. Beispielsweise können Wirkstoffformulierungen, die wenigstens einen insektiziden, akariziden oder nematiziden Wirkstoff enthalten, zur Bekämpfung von schädlichen Arthropoden, z. B. Insekten oder Akariden oder Nematoden eingesetzt werden. Wenn die erfindungsgemäßen Wirkstoffformulierungen wenigstens einen fungiziden Wirkstoff enthalten, können sie zur Bekämpfung von Schadpilzen eingesetzt werden. Wenn die erfindungsgemäßen Wirkstoffformulierungen einen herbiziden Wirkstoff enthalten, können sie zur Bekämpfung von Ungräsern und dergleichen eingesetzt werden.

Je nach Art des Wirkstoffs werden die erfindungsgemäßen Zusammensetzungen insbesondere zum Schutz von Pflanzen vor einem Befall mit Schadorganismen wie Insekten, Akariden, Nematoden, oder zum Schutz vor einem Befall mit pflanzenpathogenen Pilzen und dergleichen, oder bei der Saatgutbehandlung oder im Materialschutz eingesetzt, beispielsweise zum Schutz von Lignocellulosematerialien wie Holz, vor einem Befall mit Schadinsekten, wie holzzerstörenden Käfern, Termiten, Ameisen und dergleichen, oder vor einem Befall mit holzverfärbenden oder holzzerstörenden Pilzen.

Selbstverständlich können die erfindungsgemäßen Zusammensetzungen auch in der Kosmetik oder in der Medizin oder in technischen Anwendungen eingesetzt werden.

Die folgenden Beispiele dienen der Erläuterung der Erfindung und sind nicht einschränkend zu verstehen.

### Analytik:

Die Bestimmung der Glasübergangstemperatur erfolgte mittels eines DSC-Gerätes DSC30 der Fa. Mettler bei einer Aufheizrate von 10 K/min.

Die Bestimmung der Molekulargewichte erfolgte mittels Gelpermeationschromatographie (Gerät "Series 1100" der Fa. Agilent) unter Verwendung eines RI-Detektors und einer 5µ Mixed-D-Säule der Fa. PL bei 30°C (Säulentemperatur). Als Eluent diente Dimethylformamid, das 0,5 % Lithiumbromid enthielt. Die Flussrate betrug 1 ml/min. Die Eichung erfolgte mittels Polymethylmethacrylat-Eichsätzen.

### Herstellung der Kammpolymere

In den Herstellungsbeispielen 1 bis 6 wurde ein Ester der Methacrylsäure mit einem Methylpolyethylenglykol (im Folgenden Polyethylenglykolmethylethermethacrylat) eingesetzt, wobei der Ester ein Molekulargewicht (Zahlenmittel) von 1100 Dalton aufwies. Dies entspricht im Mittel etwa 23 Ethylenoxid-Wiederholungseinheiten in der Polyetherkette.

### Herstellungsbeispiele 1-3

### Allgemeine Herstellungsvorschrift 1:

In einem Synthesereaktor AutoPlant A100 der Fa. Chemspeed^{®} wurden je Reaktionsgefäß 15 ml Dimethylformamid (DMF) vorgelegt und auf 95 °C erwärmt. Hierzu wurden unter Rühren und Beibehaltung der Temperatur parallel innerhalb von 180 min Zulauf 1, sowie - zeitgleich mit Zulauf 1 startend - innerhalb von 195 min Zulauf 2 zudosiert. Nach Beendigung der Zuläufe wurde weitere 60 min bei 95 °C nachpolymerisiert.

### Herstellungsbeispiel 1: Kammpolymer K1

Zulauf 1: Mischung bestehend aus 7,0 g Methylmethacrylat, 3,5 g n-Butylacrylat und 10,5 g Polyethylenglykolmethylethermethacrylat, gelöst in DMF auf 49 ml. Zulauf 2: 0,63 g 2,2'-Azobis(2-methylpropionitril) gelöst in DMF auf 6 ml.

### Herstellungsbeispiel 2: Kammpolymer K2

Zulauf 1: Mischung bestehend aus 3,5 g Laurylacrylat, 7,0 g Methylmethacrylat und 10,5 g Polyethylenglykolmethylethermethacrylat, gelöst in DMF auf 49 ml. Zulauf 2: 0,63 g 2,2'-Azobis(2-methylpropionitril) gelöst in DMF auf 6 ml.

### Herstellungsbeispiel 3: Kammpolymer K3

Zulauf 1: Mischung bestehend 14,0 g Methylmethacrylat und 7,0 g Polyethylenglykolmethylethermethacrylat, gelöst in DMF auf 49 ml. Zulauf 2: 0, 63 g 2,2'-Azobis(2-methylpropionitril) gelöst in DMF auf 6 ml.

### Herstellungsbeispiele 4 bis 6

### Allgemeine Herstellungsvorschrift 2:

In einem Synthesereaktor Accelerator™ SLT100 der Fa. Chemspeed^{®} wurde je Reaktionsgefäß 6,72 ml von Monomerlösung 1 mit 0,21 ml von Initiatorlösung 2 versetzt. Anschließend wurde der Ansatz unter Schütteln 4 h auf 95 °C erhitzt, bevor weitere 0,07 ml von Initiatorlösung 2 zugegeben und die Polymerisation innerhalb von 2 h bei 95 °C abgeschlossen wurde.

### Herstellungsbeispiel 4: Kammpolymer K4

Monomerlösung 1: Mischung bestehend aus 300 mg Methylmethacrylat, 150 mg 1-Vinyl-2-pyrrolidon und 450 mg Polyethylenglykolmethylethermethacrylat gelöst in DMF auf 8,64 ml.

Initiatorlösung 2:100 mg 2,2'-Azobis(2-methylpropionitril) gelöst in DMF auf 1,00 ml.

### Herstellungsbeispiel 5: Kammpolymer K5

Monomerlösung 1: Mischung bestehend aus 300 mg Methylmethacrylat, 300 mg N-Vinylcaprolactam und 300 mg Polyethylenglykolmethylethermethacrylat gelöst in DMF auf 8,64 ml.

Initiatorlösung 2: 100 mg 2,2'-Azobis(2-methylpropionitril) gelöst in DMF auf 1,00 ml.

### Herstellungsbeispiel 6: Kammpolymer K6

Monomerlösung 1: Mischung bestehend aus 150 mg Acrylsäure 450 mg 2-Phenoxyethylacrylat und 300 mg Polyethylenglykolmethylethermethacrylat gelöst in DMF auf 8,64 ml.

Initiatorlösung 2: 100 mg 2,2'-Azobis(2-methylpropionitril) gelöst in DMF auf 1,00 ml.

Die in Tabelle 1 zusammengestellten Beispiele der Kammpolymere K7 bis K11 wurden unter Verwendung der Herstellungsvorschriften 1 erhalten.

**Tabelle 1:**

| Bsp. | Monomer Ma | | Monomer Mb | | Monomer Mc | | M_{w} |
|---|---|---|---|---|---|---|---|
| | Typ | Menge | Typ | Menge | Typ | Menge | |
| K7 | B | 50 | A | 50 | - | - | |
| K8 | B | 67 | A | 33 | - | - | |
| K9 | C | 50 | A | 33 | D | 17 | |
| K10 | B | 50 | A | 33 | D | 17 | |
| K11 | B | 33 | A | 50 | D | 17 | |

Die Mengenangabe erfolgt in Gew.-% bezogen auf die Gesamtmenge an Monomeren M
- A: Polyethylenglykolmonoethylethermethacrylat
- B: Methylmethacrylat
- C: Butylacrylat
- D: Acrylsäure

### Herstellung der erfindungsgemäßen Wirkstoffformulierungen

### Allgemeine Herstellungsvorschrift für feste Wirkstoffformulierungen

In einer Lösung des Kammpolymers (30 g) in DMF (70 g) wurde der Wirkstoff oder das Wirkstoffgemisch (Gesamtmenge 10 g) gelöst. Man entfernte das Lösungsmittel im Vakuum bei einer Temperatur von 80°C, wobei man eine feste homogene Masse erhielt, die keine kristallinen Bestandteile aufwies.

Die in Tabelle 2 zusammengestellten Wirkstoffformulierungen wurden unter Verwendung der allgemeinen Herstellungsvorschrift bereitgestellt.

**Tabelle 2:**

| Beispiel | Wirkstoff 1 | Menge | Wirkstoff 2 | Menge | Polymer | Menge |
|---|---|---|---|---|---|---|
| Z1 | Pyraclostrobin | 15 | Epoxiconazol | 10 | K7 | 75 |
| Z2 | Pyraclostrobin | 15 | Epoxiconazol | 10 | K8 | 75 |
| Z3 | Pyraclostrobin | 15 | Epoxiconazol | 10 | K9 | 75 |
| Z4 | Pyraclostrobin | 15 | Epoxiconazol | 10 | K10 | 75 |
| Z5 | Pyraclostrobin | 15 | Epoxiconazol | 10. | K11 | 75 |
| Z6 | Metconazol | 25 | - | - | K7 | 75 |
| Z7 | Metconazol | 25 | - | - | K8 | 75 |
| Z8 | Metconazol | 25 | - | - | K9 | 75 |
| Z9 | Metconazol | 25 | - | - | K10 | 75 |
| Z10 | Metconazol | 25 | - | - | K11 | 75 |
| Z11 | Epoxiconazol | 25 | - | - | K7 | 75 |
| Z12 | Epoxiconazol | 25 | - | - | K8 | 75 |
| Z13 | Epoxiconazol | 25 | - | - | K9 | 75 |
| Z14 | Epoxiconazol | 25 | - | - | K10 | 75 |
| Z15 | Epoxiconazol | 25 | - | - | K11 | 75 |
| Z16 | Boscalid | 25 | - | - | K7 | 75 |
| Z17 | Boscalid | 25 | - | - | K8 | 75 |
| Z18 | Boscalid | 25 | - | - | K9 | 75 |
| Z19 | Boscalid | 25 | - | - | K10 | 75 |
| Z20 | Boscalid | 25 | - | - | K11 | 75 |
| Z21 | Pyraclostrobin | 15 | Metconazol | 10 | K7 | 75 |
| Z22 | Pyraclostrobin | 15 | Metconazol | 10 | K8 | 75 |
| Z23 | Pyraclostrobin | 15 | Metconazol | 10 | K9 | 75 |
| Z24 | Pyraclostrobin | 15 | Metconazol | 10 | K10 | 75 |
| Z25 | Pyraclostrobin | 15 | Metconazol | 10 | K11 | 75 |
| Z26 | Epoxiconazol | 14 | Metconazol | 11 | K7 | 75 |
| Z27 | Epoxiconazol | 14 | Metconazol | 11 | K8 | 75 |
| Z28 | Epoxiconazol | 14 | Metconazol | 11 | K9 | 75 |
| Z29 | Epoxiconazol | 14 | Metconazol | 11 | K10 | 75 |
| Z30 | Epoxiconazol | 14 | Metconazol | 11 | K11 | 75 |
| Z31 | CMPTP | 25 | - | - | K7 | 75 |
| Z32 | CMPTP | 25 | - | - | K8 | 75 |
| Z33 | CMPTP | 25 | - | - | K9 | 75 |
| Z34 | CMPTP | 25 | - | - | K10 | 75 |
| Z35 | CMPTP | 25 | - | - | K11 | 75 |
| Z36 | CMPTP | 14 | Epoxiconazol | 11 | K7 | 75 |
| Z37 | CMPTP | 14 | Epoxiconazol | 11 | K8 | 75 |
| Z38 | CMPTP | 14 | Epoxiconazol | 11 | K9 | 75 |
| Z39 | CMPTP | 14 | Epoxiconazol | 11 | K10 | 75 |
| Z40 | CMPTP | 14 | Epoxiconazol | 11 | K11 | 75 |

Die Mengenangabe erfolgt in Gew.-% bezogen auf die Summe der Mengen an Wirkstoff und Polymer.
CMPTP 5-Chlor-6-(2,4,6-trifluorphenyl)-7-(4-methylpiperidin-1-yl)-1,2,4-triazolo-[1,5-a]pyrimidin

Beim Verdünnen der Zubereitungen Z1 bis Z40 mit Wasser auf eine Wirkstoffkonzentration von 64 ppm erhielt man optisch wasserklare Lösungen. Die mittlere Teilchengröße aller Proben lag demnach unterhalb 100 nm.

### Anwendungstechnische Prüfung.

### Untersuchung der fungiziden Wirkung

Die jeweilige Wirkstoffformulierungen wurde als Stammlösung mit einer Konzentration von 64 ppm Wirkstoff aufbereitet und anschließend mit Wasser zu der unten angegeben Wirkstoffkonzentration verdünnt (Tabelle 3).

Blätter von in Töpfen gewachsenen Weizensämlingen der Sorte "Kanzler" wurden mit einer Sporensuspension des Braunrostes (Puccinia recondita) inokuliert. Danach wurden die Töpfe für 24 Stunden in eine Kammer mit hoher Luftfeuchtigkeit (90 bis 95 %) und 20 bis 22 °C gestellt. Während dieser Zeit keimten die Sporen aus und die Keimschläuche drangen in das Blattgewebe ein. Die infizierten Pflanzen wurden am nächsten Tag mit einer wässrigen Suspension in der unten angegebenen Wirkstoffkonzentration bis zur Tropfnässe besprüht. Die Suspension wurde wie oben beschrieben hergestellt. Nach dem Antrocknen des Spritzbelages wurden die Versuchspflanzen im Gewächshaus bei Temperaturen zwischen 20 und 22 °C und 65 bis 70 % relativer Luftfeuchte für 7 Tage kultiviert. Dann wurde das Ausmaß der Rostpilzentwicklung auf den Blättern ermittelt.

Die Ergebnisse der biologischen Prüfung sind in Tabelle 3 zusammengefasst. Die Ergebnisse zeigen, dass die mit den Kammpolymeren stabilisierte Wirkstoffmischung bei geringen Aufwandmengen eine bessere fungizide Aktivität als kommerzielle Produkte aufweist.

**Tabelle 3:**

| | Befall [%] | Befall [%] | Befall [%] | Befall [%] | Befall [%] | Befall [%] |
|---|---|---|---|---|---|---|
| Zubereitung: Aufwandmenge [ppm] | Z1 | Z2 | Z3 | Z4 | Z5 | konv. Suspo-emulsion ¹⁾ |
| 63 | 0 | 1 | 0 | 0 | 0 | 3 |
| 32 | 0 | 7 | 4 | 3 | 3 | 17 |
| 16 | 6 | 17 | 10 | 20 | 17 | 50 |

| | | | | | | |
|---|---|---|---|---|---|---|
| 1) Zubereitung der konventionellen Suspoemulsion: 4,7 Gew.-% Epoxiconazol 12,5 Gew.-% Pyraclostrobin 29,2 Gew.-% aromatische Lösungsmittel ca. 12 Gew.-% Fettalkoholethoxilat ca. 4 Gew.-% Phenolsulfonsäure-Formaldehyd-Kondensat-Natriumsalz Verdicker Biozid in 1 I wässriger Formulierung | | | | | | |

## Patentansprüche

1. Wirkstoffzusammensetzung, enthaltend
i) wenigstens ein Kammpolymer und
ii) wenigstens einen organischen Wirkstoff aus der Gruppe der Wirkstoffe für den Pflanzenschutz und pharmazeutischen Wirkstoffen, wobei der Wirkstoff in Wasser bei 25 °C/1013 mbar eine Löslichkeit unterhalb 10 g/l aufweist,
wobei das Kammpolymer erhältlich ist durch Copolymerisation monoethylenisch ungesättigter Monomere M, umfassend:
a) wenigstens ein monoethylenisch ungesättigtes Monomer Ma, ausgewählt unter Estern monoethylenisch ungesättigter C₃-C₈monocarbonsäuren mit C₁-C₂₀Alkanolen, C₅-C₁₀-Cycloalkanolen, Phenyl-C₁-C₄-alkanolen oder Phenoxy-C₁₋C₄-alkanolen und den Diestern monoethylenisch ungesättigter C₄-C₈-Dicarbonsäuren mit C₁-C₂₀-Alkanolen, C₅-C₁₀-Cycloalkanolen, Phenyl-C₁-C₄-alkanolen oder Phenoxy-C₁-C₄-alkanolen;
b) wenigstens ein monoethylenisch ungesättigtes Monomer Mb, ausgewählt unter den Estern monoethylenisch ungesättigter C₃-C₈-Monocarbonsäuren und den Mono- und Diestern monoethylenisch ungesättigter C₄-C₈-Dicarbonsäuren mit Poly-(C₂-C₄-alkylenether)olen;
c) ein monoethylenisch ungesättigtes Monomer Mc, das von den Monomeren Ma und Mb verschieden ist;
wobei die Gesamtmenge der Monomere Ma und Mb wenigstens 80 Gew.-% der das Kammpolymer konstituierenden Monomere M ausmacht, und wobei in dem Kammpolymer das Monomer Mc 5 bis 20 Gew.-%, bezogen auf die Gesamtmenge der Monomere M ausmacht.

2. Wirkstoffzusammensetzung nach Anspruch 1, wobei in dem Kammpolymer wenigstens 50 Gew.-% der die Poly-(C₂C₄-alkylenether)ole bildenden Wiederholungseinheiten die Formel CH₂CH₂O aufweisen.

3. Wirkstofftusammensetzung nach Anspruch 1 oder 2, wobei in dem Kammpolymer die Poly-(C₂-C₄-alkylenether)ole ein Molekulargewicht im Bereich von 200 bis 2000 aufweisen.

4. Wirkstoffzusammensetzung nach einem der vorhergehenden Ansprüche, wobei in dem Kammpolymer das Poly-(C₂-C₄-alkylenether)ol ein Poly-C₂-C₄-alkylenglykol-mono-C₁-C₁₀-alkylether Ist.

5. Wirkstoffzusammensetzung nach einem der vorhergehenden Ansprüche, wobei in dem Kammpolymer das wenigstens eine Monomer Mb ausgewählt ist unter Estern der Acrylsäure und der Methacrylsäure mit Poly-(C₂-C₄-alkylenether)olen.

6. Wirkstoffzusammensetzung nach einem der vorhergehenden Ansprüche, wobei in dem Kammpolymer das wenigstens eine Monomer Ma ausgewählt ist unter Estern der Acrylsäure und der Methacrylsäure mit C₁-C₂₀-Alkanolen, C₅-C₁₀-Cycloalkanolen, Phenyl-C₂-C₄-alkanolen oder Phenoxy-C₁-C₄-alkanolen.

7. Wirkstoffzusammensetzung nach einem der vorhergehenden Ansprüche, wobei in dem Kammpolymer das wenigstens eine Monomer Mb 10 bis 90 Gew.%, bezogen auf die Gesamtmenge der Monomere M ausmacht.

8. Wirkstoffzusammensetzung nach einem der vorhergehenden Ansprüche, wobei in dem Kammpolymer das wenigstens eine Monomer Ma 10 bis 90 Gew.-%, bezogen auf die Gesamtmenge der Monomere M ausmacht.

9. Wlrkstoffzusammensetzung nach einem der vorhergehenden Ansprüche, wobei In dem Kammpolymer die Monomere M zusätzlich ein oder mehrere Monomere Mc mit einer Wasserlöslichkeit oberhalb 60 g/l bei 20°C umfassen.

10. Wirkstoffzusammensetzung nach einem der vorhergehenden Ansprüche, wobei das Kammpolymer ein zahlenmittleres Molekulargewicht im Bereich von 2000 bis 500000 Dalton aufweist.

11. Wirkstoffzusammensetzung nach einem der vorhergehenden Ansprüche, worin das Kammpolymer in einer Menge von 0,3 bis 10 Gew:-Tellen, bezogen auf 1 Gew.-Tell Wirkstoff, vorliegt.

12. Wirkstoffzusammensetzung nach einem der vorhergehenden Ansprüche, wobei der Wirkstoff ausgewählt ist unter fungiziden Wirkstoffen und Insektiziden Wirkstoffen.

13. Verfahren zur Herstellung einer Wirkstoffzusammensetzung nach einem der vorhergehenden Ansprüche, umfassend das Vermischen des wenigstens einen Wirkstoffs mit einer Lösung oder Schmelze des Kammpolymeren.

14. Verwendung von Kammpolymeren wie in einem der Ansprüche 1 bis 11 definiert zur Herstellung von wässrigen Aufbereitungen von Wirkstoffen, die In Wasser bei 25 °C/1013 mbar eine Löslichkeit unterhalb 10 g/l aufweisen, worin die Wirkstoffe Teilchengröße unterhalb 500 nm aufweisen, wobei man das Kammpolymer in einer Menge von 0,3 bis 10 Gew.-Tellen, bezogen auf 1 Gew.-Teil Wirkstoff einsetzt.

## Claims

1. An active substance composition, which comprises
i) at least one comb polymer and
ii) at least one organic active substance from the group consisting of active substances for plant protection and pharmaceutical active substances, the active substance exhibiting a solubility in water at 25°C/1013 mbar of less than 10 g/l,
the comb polymer being obtainable by copolymerization of monoethylenically unsaturated monomers M, which comprises:
a) at least one monoethylenically unsaturated monomer Ma chosen from esters of monoethylenically unsaturated C₃-C₈-monocarboxylic acids with C₁-C₂₀-alkanols, C₅-C₁₀-cycloalkanols, phenyl-C₁-C₄-alkanols or phenoxy-C₁-C₄-alkanols and the diesters of monoethylenically unsaturated C₄-C₈-dicarboxylic acids with C₁-C₂₀-alkanols, C₅-C₁₀-cycloalkanols, phenyl-C₁-C₄-alkanols or phenoxy-C₁-C₄-alkanols;
b) at least one monoethylenically unsaturated monomer Mb chosen from the esters of monoethylenically unsaturated C₃-C₈-monocarboxylic acids and the mono- and diesters of monoethylenically unsaturated C₄-C₈-dicarboxylic acids with poly(C₂-C₄-alkylene ether)ols;
c) a monoethylenically unsaturated monomer Mc which is different from the monomers Ma and Mb;
the total amount of the monomers Ma and Mb making up at least 80% by weight of the monomers M constituting the comb polymer and in the comb polymer, the monomer Mc making up 5 to 20% by weight, based on the total amount of the monomers M.

2. The active substance composition according to claim 1, in the comb polymer at least 50% by weight of the repeat units forming the poly(C₂-C₉-alkylene ether)ols exhibiting the formula CH₂CH₂O.

3. The active substance composition according to claim 1 or 2, in the comb polymer the poly(C₂-C₄-alkylene ether)ols exhibiting a molecular weight in the range from 200 to 2000.

4. The active substance composition according to any of the preceding claims, in the comb polymer the poly(C₂-C₄-alkylene ether) ol being a poly-C₂-C₄-alkylene glycol mono-C₁-C₁₀-alkyl ether.

5. The active substance composition according to any of the preceding claims, in the comb polymer the at least one monomer Mb being chosen from esters of acrylic acid and methacrylic acid with poly(C₂-C₄-alkylene ether)ols.

6. The active substance composition according to any of the preceding claims, in the comb polymer the at least one monomer Ma being chosen from esters of acrylic acid and methacrylic acid with C₁-C₂₀-alkanols, C₅-C₁₀-cycloalkanols, phenyl-C₁-C₄-alkanols or phenoxy-C₁-C₄-alkanols.

7. The active substance composition according to any of the preceding claims, in the comb polymer the at least one monomer Mb making up 10 to 90% by weight, based on the total amount of the monomers M.

8. The active substance composition according to any of the preceding claims, in the comb polymer the at least one monomer Ma making up 10 to 90% by weight, based on the total amount of the monomers M.

9. The active substance composition according to any of the preceding claims, in the comb polymer the monomers M additionally comprising one or more monomers Mc with a solubility in water of greater than 60 g/l at 20°C.

10. The active substance composition according to any of the preceding claims, the comb polymer exhibiting a number-average molecular weight ranging from 2000 to 500 000 daltons.

11. The active substance composition according to any of the preceding claims, wherein the comb polymer is present in an amount of 0.3 to 10 parts by weight, based on 1 part by weight of active substance.

12. The active substance composition according to any of the preceding claims, the active substance being chosen from fungicidal active substances and insecticidal active substances.

13. A process for the manufacture of an active substance composition according to any of the preceding claims, which comprises the mixing of the at least one active substance with a solution or melt of the comb polymer.

14. The use of a comb polymer as defined in any of claims 1 to 11 in the manufacture of aqueous preparations of active substances which exhibit a solubility in water at 25°C/1013 mbar of less than 10 g/l, wherein the active substances exhibit particle sizes of less than 500 nm, the comb polymer being used in an amount of 0.3 to 10 parts by weight, based on 1 part by weight of active substance.

## Revendications

1. Composition de substance active, contenant
i) au moins un polymère en peigne et
ii) au moins une substance active organique choisie dans le groupe des substances actives à usage phytosanitaire et des substances actives pharmaceutiques, la substance active ayant une solubilité dans l'eau à 25 °C/1013 mbars qui est inférieure à 10 g/l,
le polymère en peigne pouvant être obtenu par copolymérisation de monomères M à insaturation monoéthylénique, comprenant :
a) au moins un monomère Ma à insaturation monoéthylénique, choisi parmi des esters d'acides monocarboxyliques en C₃-C₈ à insaturation monoéthylénique avec des alcanols en C₁-C₂₀, des cycloalcanols en C₅-C₁₀, des phényl-alcanols(C₁-C₄) ou des phénoxy-alcanols(C₁-C₄) et les diesters d'acides dicarboxyliques en C₄-C₈ à insaturation monoéthylénique avec des alcanols en C₁-C₂₀, des cycloalcanols en C₅-C₁₀, des phényl-alcanols(C₁-C₄) ou des phénoxy-alcanols(C₁-C₄);
b) au moins un monomère Mb à insaturation monoéthylénique, choisi parmi les esters d'acides monocarboxyliques en C₃-C₈ à insaturation monoéthylénique et les mono- et diesters d'acides dicarboxyliques en C₄-C₈ à insaturation monoéthylénique avec des poly[alkylène(C₂-C₄)-éther]-ols ;
c) un monomère Mc à insaturation monoéthylénique, qui est différent des monomères Ma et Mb ;
la quantité totale des monomères Ma et Mb représentant au moins 80 % en poids des monomères M constituant le polymère en peigne, et le monomère Mc dans le copolymère en peigne constituant 5 à 20 % en poids, par rapport à la quantité totale des monomères M.

2. Composition de substance active selon la revendication 1, dans laquelle au moins 50 % en poids des motifs répétitifs constituant les poly[alkylène(C₂-C₄)éther]-ols dans le polymère en peigne présentent la formule CH₂CH₂O.

3. Composition de substance active selon la revendication 1 ou 2, dans laquelle les poly[alkylène(C₂-C₄)éther]-ols dans le polymère en peigne ont une masse moléculaire dans la plage de 200 à 2 000.

4. Composition de substance active selon l'une quelconque des revendications précédentes, dans laquelle le poly[alkylène(C₂-C₄)éther]-ol dans le polymère en peigne est un éther monoalkylique en C₁-C₁₀ de polyalkylène(C₂-C₄)glycol.

5. Composition de substance active selon l'une quelconque des revendications précédentes, dans laquelle ledit au moins un monomère Mb dans le polymère en peigne est choisi parmi des esters de l'acide acrylique et de l'acide méthacrylique avec des poly[alkylène(C₂-C₄)éther]-ols.

6. Composition de substance active selon l'une quelconque des revendications précédentes, dans laquelle ledit au moins un monomère Ma dans le polymère en peigne est choisi parmi des esters de l'acide acrylique et de l'acide méthacrylique avec des alcanols en C₁-C₂₀, des cycloalcanols en C₅-C₁₀, des phényl-alcanols(C₁-C₄) ou des phénoxy-alcanols(C₁-C₄).

7. Composition de substance active selon l'une quelconque des revendications précédentes, dans laquelle ledit au moins un monomère Mb dans le polymère en peigne constitue 10 à 90 % en poids, par rapport à la quantité totale des monomères M.

8. Composition de substance active selon l'une quelconque des revendications précédentes, dans laquelle ledit au moins un monomère Ma dans le polymère en peigne constitue 10 à 90 % en poids, par rapport à la quantité totale des monomères M.

9. Composition de substance active selon l'une quelconque des revendications précédentes, dans laquelle les monomères M dans le polymère en peigne comprennent en outre un ou plusieurs monomères Mc ayant une solubilité dans l'eau supérieure à 60 g/l à 20 °C.

10. Composition de substance active selon l'une quelconque des revendications précédentes, dans laquelle le polymère en peigne présente une masse moléculaire moyenne en nombre dans la plage de 2 000 à 500 000 daltons.

11. Composition de substance active selon l'une quelconque des revendications précédentes, dans laquelle le polymère en peigne est présent en une quantité de 0,3 à 10 parties en poids, par rapport à 1 partie en poids de substance active.

12. Composition de substance active selon l'une quelconque des revendications précédentes, dans laquelle la substance active est choisie parmi des substances actives fongicides et des substances actives insecticides.

13. Procédé pour la préparation d'une composition de substance active selon l'une quelconque des revendications précédentes, comprenant le mélange de ladite au moins une substance active avec une solution ou masse fondue du polymère en peigne.

14. Utilisation de polymères en peigne tels que définis dans l'une quelconque des revendications 1 à 11, pour la fabrication de préparations aqueuses de substances actives qui présentent une solubilité dans l'eau à 25 °C/1013 mbars qui est inférieure à 10 g/l, dans lesquelles les substances actives présentent des tailles de particules inférieures à 500 nm, le polymère en peigne étant utilisé en une quantité de 0,3 à 10 parties en poids, par rapport à 1 partie en poids de substance active.
